(19) Europäisches Patentamt
European Patent Office
Office européen des brevets

(11) EP 4 438 658 A1

(12) EUROPEAN PATENT APPLICATION
published in accordance with Art. 153(4) EPC

(43) Date of publication:
02.10.2024 Bulletin 2024/40

(21) Application number: 22899016.4

(22) Date of filing: 23.11.2022

(51) International Patent Classification (IPC):
*C08G 83/00* *(2006.01)* *C08F 220/32* *(2006.01)*
*A61L 27/14* *(2006.01)*

(52) Cooperative Patent Classification (CPC):
A61L 27/14; C08F 220/32; C08F 299/04;
C08G 81/02; C08G 83/00

(86) International application number:
PCT/KR2022/018588

(87) International publication number:
WO 2023/096334 (01.06.2023 Gazette 2023/22)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 23.11.2021 KR 20210162378

(71) Applicant: TMD Lab Co. Ltd
Seoul 04799 (KR)

(72) Inventors:
- PARK, Ju Young
  Seoul 04799 (KR)
- LEE, Kang Suk
  Seoul 04799 (KR)
- YI, Se Won
  Seoul 04799 (KR)
- KANG, Mi-Lan
  Seoul 04799 (KR)

(74) Representative: Weickmann & Weickmann PartmbB
Postfach 860 820
81635 München (DE)

# (54) SHAPE-MEMORY POLYMER COMPRISING 6-ARM STRUCTURE COMPOUND, AND USE THEREOF

(57) The present invention relates to a shape-memory polymer having a 6-arm structure and use thereof. The shape-memory polymer according to the present invention exhibits shape-memory properties by cross-linking, can control temperature for shape restoration through monomer control and has biocompatibility. As a material, it can be developed into a medical material such as a support for the outer wall of blood vessels.

【FIG. 1】

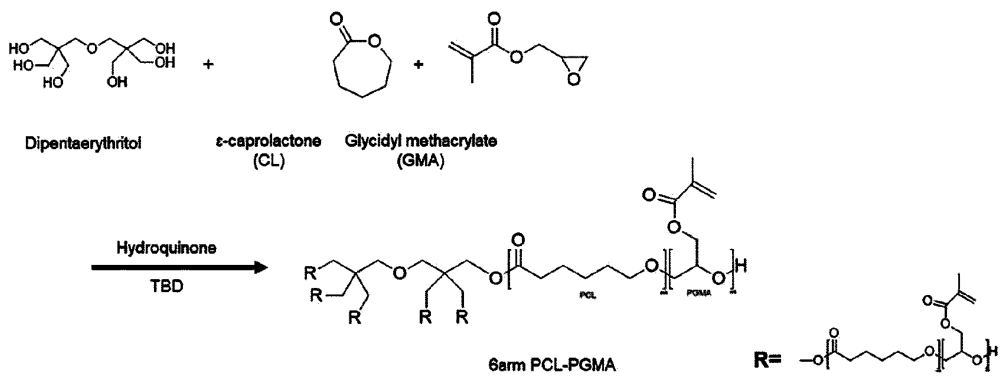




EP 4 438 658 A1

## Description

[Technical Field]

The present invention relates to a shape-memory polymer including a 6-arm structure compound and use thereof, and more specifically to a shape-memory polymer including a 6-arm structure compound that exhibits shape-memory properties by cross-linking and is controllable in shape restoration temperature through monomer control, and use thereof.

[Background Art]

Chronic renal failure is a disease in which the function of the kidneys to remove waste products is reduced and cannot be restored to normal, and treatment methods vary depending on the degree of decline in kidney function. In particular, when the stage of renal failure is severe, it is necessary to remove waste products accumulated in the blood through dialysis as an alternative therapy or to transplant a kidney. Therefore, as the number of chronic renal failure patients increases, the number of hemodialysis patients continues to increase.

Hemodialysis is a process in which blood passes through a dialysis membrane at a rate of 200 to 500 mL per minute and then returns to the patient's blood vessels, and there is a problem in that since much blood cannot be moved through peripheral blood vessels and dialysis is impossible, a dialysis channel may be formed by using artificial blood vessels using ultrasound (arteriovenous graft, AVG) depending on the patient's vascular condition at the time of surgery, but generally, hemodialysis is performed by using an arteriovenous fistula (AVF), which connects an artery and a vein. Arteriovenous fistulas usually form in the wrist or proximal upper limbs, and when arteriovenous fistula formation is performed by using autologous veins, the diameter of the veins increases due to arterial pressure, thereby causing the veins to mature, and through this, the veins are remodeled, thereby making continuous dialysis possible.

If a patient with chronic renal failure does not undergo a kidney transplant, hemodialysis through an arteriovenous fistula must be continuously performed throughout the life of the patient, and thus, blood vessel management is required for the long-term use of an arteriovenous fistula. In this case, if blood vessels are not managed properly, arteriovenous fistula complications (stenosis, thrombosis, infection, pseudoaneurysm, hemorrhage, venous pressure increase, congestive heart failure, arrhythmia syndrome, ischemic simple neuropathy, etc.) may appear, and when the complications of arteriovenous fistula frequently occur, reoperation is inevitable or the number of hospitalizations increases, resulting in a decrease in the patient's quality of life and an increase in medical expenses. Particularly, in the case of an arteriovenous fistula using an autologous vein, eddy currents are generated at the junction of the artery and vein due to the difference in physical properties between the artery and the vein and strong arterial blood, which causes the formation of a thrombus, and eventually, it causes stenosis and edema. Therefore, there is a need for a method and apparatus for preventing complications for prolonging the use of an arteriovenous fistula during dialysis.

Korean Registered Patent Nos. 10-1906472 and 10-2208921 disclose a shape-memory polymer, a preparation method thereof and the use thereof as a medical material, but there is no disclosure at all about a utilization method as a medical material that can restore shape and control temperature through monomer control and is particularly suitable for arteriovenous fistula formation.

Accordingly, the inventors of the present invention focus on these problems of the related art and made diligent efforts to improve the existing shape-memory polymer into a material that is particularly suitable for blood vessel management, and as a result, when synthesizing a compound with a 6-arm structure as in the present invention, it is possible to control the shape restoration temperature by controlling the monomer in addition to shape-memory properties due to cross-linking. In addition, when such a shape-memory polymer is utilized as a medical material, the present invention was completed by confirming that it is possible to fabricate a vascular outer wall support that can prevent blood vessels from clogging physically and biologically by wrapping blood vessels on the outside.

[Disclosure]

[Technical Problem]

An object of the present invention is to provide a novel compound having a 6-arm structure, a method for preparing the same and a use thereof.

[Technical Solution]

In order to achieve the above object, the present invention provides a compound represented by Chemical Formula (1) below:

Chemical Formula (1)

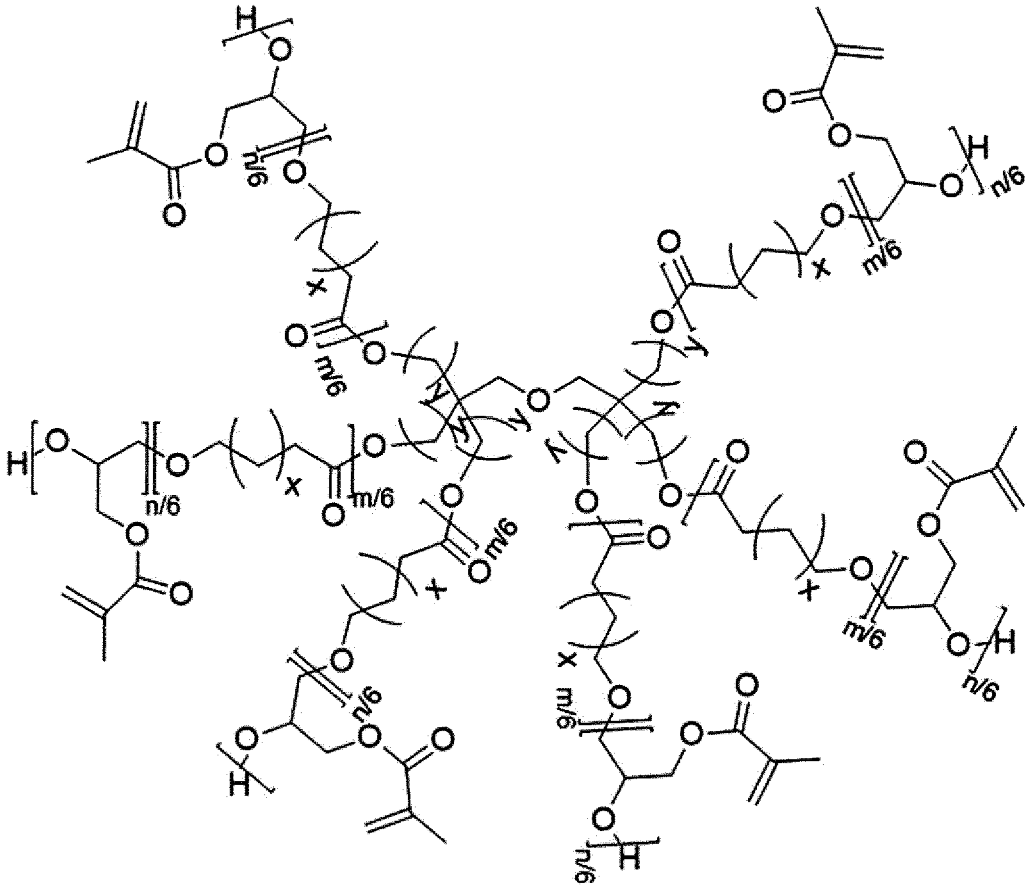

wherein in Chemical Formula (1) above, x and y are each independently integers from 1 to 20,
wherein m and n represent mol% of repeat units, and
wherein m + n is 100, and m is 70 to 99.

In the present invention, the compound may have shape-memory properties by cross-linking.

In the present invention, the compound may have an average shape restoration ability of 90% or more at a temperature of 35 to 58°C after cross-linking.

In addition, the present invention provides a method for preparing the compound of claim 1, including the step of reacting dipentaerythritol, caprolactone and glycidyl methacrylate.

In the present invention, the reaction may be a ring-opening polymerization reaction.

In the present invention, the reaction may be reacted under the presence of a catalyst selected from the group consisting of 1,5,7-triazabicyclo(4.4.0)dec-5-ene, tin(II) (2-ethylhexanoate), trimethylopropane tris(3-mercaptopropionate) and zinc succinate.

In the present invention, the method may react dipentaerythritol, caprolactone and glycidyl methacrylate at 80 to 140°C.

In addition, the present invention provides a shape-memory polymer in which the compound is cross-linked.

In addition, the present invention provides a method for preparing a shape-memory polymer, further including the step of inducing a cross-linking reaction in the compound prepared by the method.

In the present invention, the cross-linking reaction may be a photo-cross-linking or thermal cross-linking reaction.

In addition, the present invention provides a medical material, including the compound.

In the present invention, the medical material may be a support for vascular transplantation or a stent for vascular transplantation.

In the present invention, the support for vascular transplantation may be a vascular outer wall support which is provided to surround transplanted autologous veins and arteries as a side-to-end model during arteriovenous fistula formation.

In the present invention, the vascular outer wall support may be a U-shaped anastomosis device.

In the present invention, the vascular outer wall support may include an artery support and a graft vein support, and the angle of a tangent at the anastomosis site between an artery and a vein may be 30° to 90°.

[Advantageous Effects]

The 6-arm type compound according to the present invention can be prepared into a temperature-based shape-memory polymer that exhibits shape-memory properties by cross-linking, can adjust the temperature for shape restoration through monomer control, and can be developed as a medical material with biocompatibility. In particular, by cross-linking the compound of the present invention, it is possible to fabricate a vascular outer wall support which is capable of minimizing the occurrence of abnormal blood flow and smoothing the flow of blood by helping the transplanted autologous vein to mature during arteriovenous fistula formation.

[Description of Drawings]

FIG. 1 shows the synthesis mechanism of a compound having a 6-arm type structure according to some exemplary embodiments of the present invention.

FIG. 2 is a $^1$H-NMR result for the structural analysis of compounds having a 6-arm structure according to some exemplary embodiments of the present invention.

FIG. 3 is a GPC graph for the molecular weight analysis of compounds having a 6-arm type structure according to some exemplary embodiments of the present invention.

FIG. 4 is a DSC measurement graph confirming (a) the melting temperature and (b) crystallization temperature of compounds having a 6-arm structure according to some exemplary embodiments of the present invention before cross-linking to a shape-memory polymer.

FIG. 5 is a DSC measurement graph confirming (a) the melting temperature and (b) crystallization temperature of compounds having a 6-arm structure according to some exemplary embodiments of the present invention after cross-linking to a shape-memory polymer.

FIG. 6 is a graph analyzing the mechanical properties after cross-linking of shape-memory polymers according to some exemplary embodiments of the present invention through UTM measurement.

FIG. 7 is the results of analyzing the shape-memory properties after cross-linking of the shape-memory polymer according to some exemplary embodiments of the present invention through DMA measurement.

FIG. 8 is a set of photographs showing shape-memory properties after cross-linking of the shape-memory polymer according to some exemplary embodiments of the present invention.

FIG. 9 shows a mimetic diagram of a shape-memory vascular outer wall support device and the arrangement of an artery and a vein.

FIG. 10 is the results of analyzing blood flow changes according to the anastomosis angle of a vein, FIG. 10a shows the analysis results of toe, heel and floor areas of arteriovenous fistula modeling, and FIG. 10b shows blood flow analysis results in a U-shape model.

FIG. 11 is a mimetic diagram of a vascular outer wall support according to some exemplary embodiments of the present invention, FIG. 11a shows a side view of the vascular outer wall support, and FIG. 11b shows a bottom view of the vascular outer wall support.

FIG. 12 shows a vascular outer wall support which is fabricated by a shape-memory polymer according to some exemplary embodiments of the present invention.

FIG. 13 is the results of comparing the circumferential tensile strength of the vascular outer wall support which is fabricated by the shape-memory polymer according to some embodiments of the present invention with the circumferential tensile strength of a vascular outer wall support which is fabricated by a shape-memory polymer that is cross-linked with a compound having a 4-arm structure.

FIG. 14 shows (a) a measurement device for measuring a pressurized bursting strength, and (b) the results of comparing the pressurized bursting strength of the vascular outer wall support which is fabricated by the shape-memory polymer according to some embodiments of the present invention with the pressurized bursting strength of a vascular outer wall support which is fabricated by a shape-memory polymer that is cross-linked with a compound having a 4-arm structure.

FIG. 15 is a computer modeling result showing changes in the structure of a vein when the vascular outer wall support which is fabricated by a shape-memory polymer according to some exemplary embodiments of the present invention is wrapped around a blood vessel wall.

FIG. 16 shows an arteriovenous fistula animal model which is prepared through the end-to-side anastomosis of the femoral artery and femoral vein of a dog.

FIG. 17 is the results of confirming the patency and blood flow pattern according to the presence or absence of the vascular outer wall support which is fabricated by the shape-memory polymer according to some exemplary embodiments of the present invention at 3 months and 6 months after vascular anastomosis by Doppler ultrasound examination.

FIG. 18 is the results of confirming the anastomosis shape and patency by angiography according to the presence or absence of the vascular outer wall support which is fabricated by a shape-memory polymer according to some exemplary embodiments of the present invention at 6 months after angioplasty.

FIG. 19 is the results of histopathological analysis of veins at an anastomotic site according to the presence or absence of a vascular outer wall support which is fabricated by a shape-memory polymer according to some exemplary embodiments of the present invention at 6 months after angioplasty. Black - elastic fibers and nuclei, Yellow - collagen, Soft green - mucin, Red - muscle

[Modes of the Invention]

Unless defined otherwise, all technical and scientific terms used herein have the same meanings as commonly understood by one of ordinary skill in the art to which the present invention pertains. In general, the nomenclature used herein and the experimental methods described below are those that are well-known and commonly used in the art.

As used herein, the term "shape-memory polymer (SMP)" refers to a polymer that when an object is made to have a certain shape under specific conditions, even if the shape is changed by external impact thereafter, the polymer has the property of returning to the original shape when the object is kept under the same conditions as the first time (temperature, light, pH, humidity, etc.).

In the present invention, terms such as "include" or "have" are intended to designate that there is a feature, number, step, operation, component, part or combination thereof described in the specification, but it should be understood that it does not exclude in advance the possibility of the presence or addition of one or more other features, numbers, steps, operations, components, parts or combinations thereof.

In the present invention, when ε-caprolactone (ε-CL) and glycidyl methacrylate (GMA) are reacted together with dipentaerythritol, a novel polycaprolactone-based compound could be synthesized, and it was confirmed that excellent shape-memory properties were imparted when a network was formed between polymer chains of the compound through cross-linking, and it was possible to control the shape restoration temperature when controlling the monomers.

Accordingly, in one aspect, the present invention relates to a compound represented by Chemical Formula (1):

Chemical Formula (1)

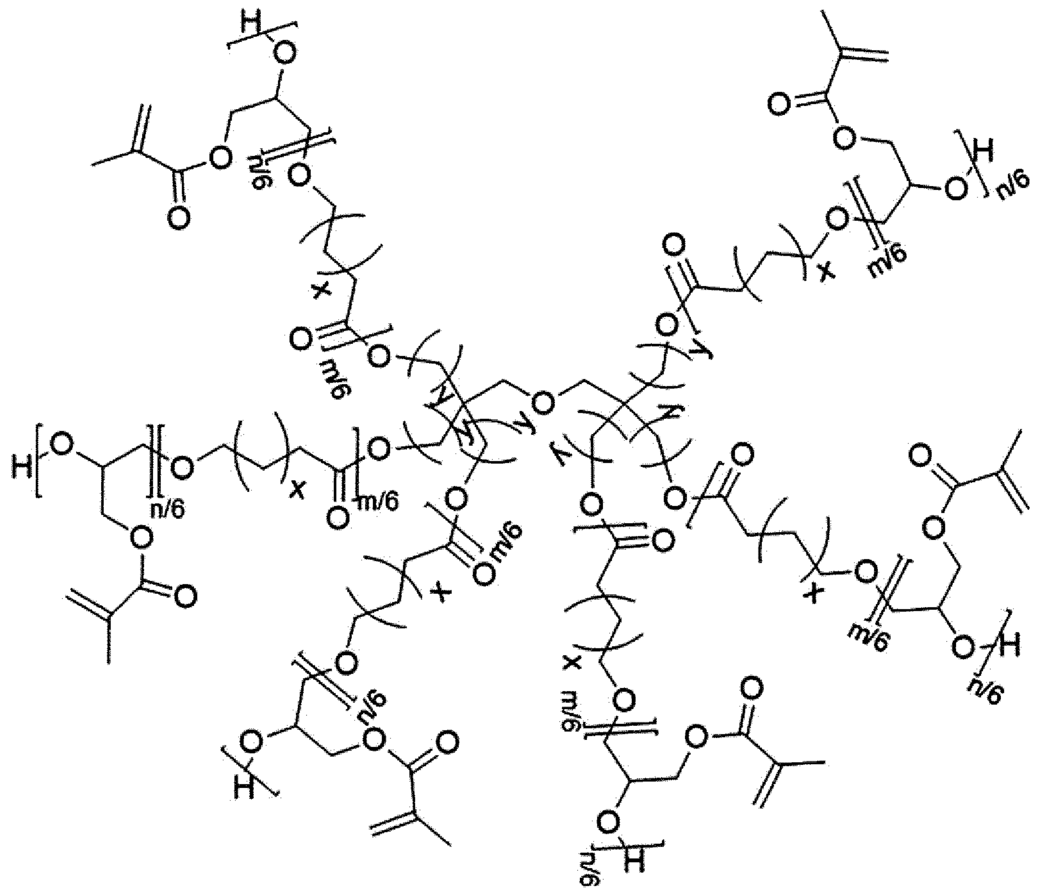

wherein in Chemical Formula (1) above, x and y are each independently integers from 1 to 20,
wherein m and n represent mol% of repeat units, and
wherein m + n is 100, and m is 70 to 99.

The shape restoration temperature of the compound of the present invention may be controlled according to the amounts of ε-caprolactone monomer and glycidyl methacrylate monomer constituting the compound.

More specifically, in Chemical Formula (1), x and y may be each independently integers of 1 to 20, which can be controlled by the carbon numbers of a lactone-based monomer and an initiator in the step of synthesizing the compound of Chemical Formula (1).

For example, x may be 3 when ε-caprolactone (ε-CL) is used, and y may be 1 when dipentaerythritol is used. In addition, the number of x may be adjusted by using monomers such as α-acetolactone, β-propiolactone, γ-butyrolactone and δ-valerolactone by replacing ε-caprolactone (ε-CL), and the number of y may be adjusted by using an initiator such as 6arm PEG in place of dipentaerythritol. In the present invention, x and y may be easily adjusted by a person skilled in the art.

In addition, in Chemical Formula (1), m and n may represent mol% of the repeating unit, m + n may be 100, and x may be 70 to 99, or 88 to 96, or 92 to 96, or 94 to 96.

Herein, the mol% means the ratio of m and n repeating units, and it may specifically mean the mole fraction (ratio). For example, in PCL-co-PGMA, it may mean the mole fraction of repeating units of PCL and PGMA.

In the present invention, the compound may be characterized in that it has shape-memory properties by cross-linking.

In the present invention, after cross-linking, the compound may have an average shape restoration ability of at least 90%, such as about 90%, about 91%, about 92%, about 93%, about 94%, about 95%, about 96%, about 97%, about 98%, about 99% or about 100% at a temperature of 35 to 58°C, or any range of 35 to 58°C, or any temperature of 35 to 58°C, such as about 35°C, about 36°C, about 37°C, about 38°C, about 39°C, about 40°C, about 41°C, about 42°C, about 43°C, about 44°C, about 45°C, about 46°C, about 47°C, about 48°C, about 49°C, about 50°C, about 51°C, about 52°C, about 53°C, about 54°C, about 55°C, about 56°C, about 57°C or about 58°C, but the present invention is

not limited thereto.

The compound of the present invention may be prepared by reacting α-acetolactone, β-propiolactone, γ-butyrolactone, δ-valerolactone or ε- caprolactone monomer together with an acrylic monomer including a glycidyl group and an initiator.

For example, the compound of the present invention may be prepared by ring-opening polymerization of dipentaerythritol, caprolactone and glycidyl methacrylate.

In this case, the reaction between temperature-sensitive glycidyl methacrylate groups may be suppressed by adding a catalyst or adding a polymerization inhibitor together with or simultaneously with an initiator during the initial reaction with little polymerization conversion, thereby improving reactivity.

Accordingly, in another aspect, the present invention relates to a method for preparing the compound, including the step of reacting dipentaerythritol, caprolactone and glycidyl methacrylate.

In the present invention, the reaction may be characterized in that it is a ring-opening polymerization reaction.

In the present invention, the reaction may be reacted under the presence of a catalyst selected from the group consisting of 1,5,7-triazabicyclo(4.4.0)dec-5-ene, tin(II) (2-ethylhexanoate), trimethylopropane tris(3-mercaptopropionate) and zinc succinate, but the present invention is not limited thereto.

In particular, as a material for inducing the simultaneous ring-opening polymerization of two monomers (CL, GMA), it is preferable to use 1,5,7-triazabicyclo(4.4.0)dec-5-ene, which can shorten the synthesis time of the compound, as a catalyst.

In the present invention, the reaction between methacrylate groups may be suppressed by introducing an initiator and/or a polymerization inhibitor during the initial reaction, that is, before introducing glycidyl methacrylate.

In addition to the above, the polymerization inhibitor serves to terminate the reaction by suppressing the exothermic reaction locally occurring in the latter half of the polymerization and removing unreacted residual radicals.

In this way, when the initiator and the polymerization inhibitor are reacted with the monomers, such caprolactone and glycidyl methacrylate, at about 110°C for about 6 hours, the ring structure in the monomer is opened, and a polycaprolactone-polyglycidyl methacrylate (6arm PCL-PGMA) copolymer with 6 arms is synthesized.

In the present invention, the initiator may be characterized in that it is dipentaerythritol, and specifically, the present invention may be characterized in that a polycaprolactone-polyglycidyl methacrylate (6-arm PCL-PGMA) copolymer having 6 arms is synthesized by initial addition of the initiator.

In the present invention, for the polymerization inhibitor, at least one selected from the group consisting of hydroquinone, hydroquinone monomethyl ether, p-benzoquinone and phenothiazine may be used, but the present invention is not limited thereto. Preferably, the polymerization inhibitor may be hydroquinone.

In the present invention, the preparation method may react dipentaerythritol, caprolactone and glycidyl methacrylate at 80 to 140°C, and preferably, 100 to 130°C, such as at about 110°C.

In this case, when the synthesis of the compound of the present invention proceeds at a temperature lower than 100°C, the catalytic reaction may not proceed, and when the synthesis of the compound of the present invention proceeds at a temperature exceeding 130°C, there may be a problem in that the catalytic reaction rate decreases.

In a preferred aspect, the polymerization mechanism of the compound of the present invention may be expressed as follows.

[Reaction Scheme 1]

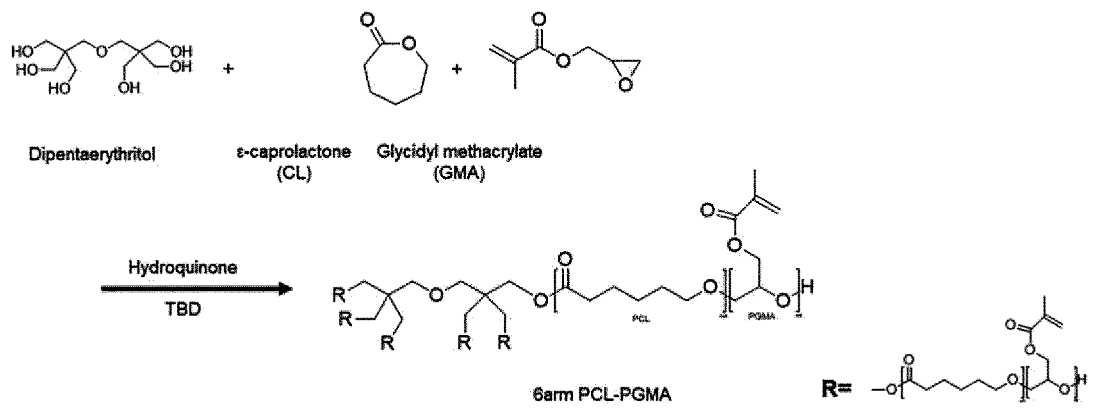


In the present invention, the cross-linking reaction may be a photo-cross-linking or thermal cross-linking reaction, but the present invention is not limited thereto.

The compound of the present invention may have a copolymer structure in which a ε-caprolactone monomer and an acrylic monomer including a glycidyl group are polymerized. For example, the compound may have a structure of a copolymer [PCL-co-PGMA) obtained by polymerizing ε-caprolactone (CL) and glycidyl methacrylate (GMA) monomers.

In the compound of the present invention, the arrangement order of the ε-caprolactone monomer and the glycidyl methacrylate monomer is not particularly limited, and they may be arranged alternately, randomly or in blocks.

In addition, a hydroxyl group or the like may be bonded to the terminal of the copolymer. Such a copolymer having a hydroxyl group attached to the terminal may be prepared by polymerization using an initiator having a hydroxyl group attached to the terminal.

Meanwhile, the glycidyl group included in the glycidyl methacrylate monomer may be a cross-linkable functional group, a photo-cross-linkable functional group or a thermally cross-linkable functional group. In addition, the copolymer may have shape-memory properties by cross-linking.

Accordingly, in another aspect, the present invention relates to a shape-memory polymer which is cross-linked with the compound.

In another aspect, the present invention relates to a method for preparing a shape-memory polymer, further including the step of inducing a cross-linking reaction in the compound.

In the present invention, the cross-linking agent for the cross-linking reaction may be at least one selected from the group consisting of potassium persulfate, ammonium persulfate, benzoyl peroxide, diauryl peroxide, dicumyl peroxide, hydrogen peroxide, azobisisobutuyronitrile, Irgacure, Darocure, LAP (lithium phenyl-2,4,6-trim ethylbenzoylphosphinate), TPO (diphenyl(2,4,6-trimethylbenzoyl)phosphine) and TPO-L (ethyl(2,4,6-trimethylbenzoyl)phenylphosphinate).

For example, the cross-linking agent that initiates the cross-linking reaction may be a peroxide cross-linking agent (benzoyl peroxide, dicumyl peroxide, etc.), Irgacure2959, Irgacure784, Irgacure819, Darocur1173 or Darocur4265, but the present invention is not limited thereto.

Meanwhile, the compound of the present invention is a temperature-sensitive compound that has biocompatibility and is capable of adjusting the shape restoration temperature through monomer control, and when a compound with a 6-arm structure was cross-linked to fabricate a shape-memory polymer compared to a compound with a 4-arm structure, it was confirmed that the circumferential tensile strength and pressurized bursting strength were improved. Actually, as a result of performing arteriovenous fistula formation by fabricating a vascular outer wall support using the compound according to the present invention, it was confirmed that the vascular U shape was well maintained, eddy current formation was suppressed after blood vessel anastomosis, and arterial blood flow was smooth, resulting in excellent patency. In addition, through histological analysis, it was confirmed that vascular stenosis can be suppressed through the formation of new vascular intima.

Therefore, in still another aspect, the present invention relates to a medical material including the compound.

In the present invention, the medical material may be prepared by using a shape-memory polymer that is cross-linked with the compound.

In the present invention, the medical material may be a support for blood vessel transplantation or a stent for blood vessel transplantation, but the present invention is not limited thereto.

In the present invention, the support for blood vessel transplantation may be characterized in that it is a vascular outer wall support which is provided to surround and arrange an autologous vein and artery transplanted in a side-to-end model during arteriovenous fistula formation, but the present invention is not limited thereto.

Such a vascular outer wall support has the properties of helping mature the transplanted autologous vein to minimize the occurrence of abnormal blood flow and facilitating the flow of blood flow.

In the present invention, the vascular outer wall support may be characterized in that it is a U-shaped anastomosis device, but the present invention is not limited thereto.

The U-shaped anastomosis device according to the present invention has the advantage of minimizing the occurrence of abnormal blood flow in blood vessels flowing from arterial blood through anastomosis to venous blood and maintaining blood flow.

In the present invention, the vascular outer wall support may include an artery support and a transplanted vein support.

In the present invention, the angle of a tangent at the anastomosis site of an artery and a vein may be 30 to 90°, and preferably 30 to 60°, or any range of 30 to 60°, such as any value of 30 to 45°, or 30 to 60°, such as about 31°, about 32°, about 33°, about 34°, about 35°, about 36°, about 37°, about 38°, about 39°, about 40°, about 41°, about 42°, about 43°, about 44°, about 45°, about 46°, about 47°, about 48°, about 49°, about 50°, about 51°, about 52°, about 53°, about 54°, about 55°, about 56°, about 57°, about 58°, about 59° or about 60°.

Adjusting the tangent angle of the anastomosis site according to the present invention has the advantages of preventing neointimal formation and preventing vascular stenosis.

In the present invention, as the curvature is lower, the blood flow may become more stable, but considering the anatomical structure, the angle of a tangent at the anastomosis site of an artery and a vein may be most preferably

30 to 45°, but the present invention is not limited thereto.

## [Example]

Hereinafter, the present invention will be described in more detail through examples. These examples are only for illustrating the present invention, and it will be apparent to those skilled in the art that the scope of the present invention is not to be construed as being limited by these examples.

### Example: Synthesis of 6-arm PCL-PGMA

By varying the ratio of PCL and PGMA, the following 6-arm PCL-PGMA copolymer was synthesized.

**[Table 1]**

| **Example** | **Dipentaerythritol (mmol)** | **HQ (mmol)** | **ε-CL (mmol)** | **GMA (mmol)** | **TBD (mmol)** |
|---|---|---|---|---|---|
| Example 1 (6 arm 92% PCL-08% PGMA) | 0.5 | 4.9 | 301 | 49 | 1 |
| Example 2 (6 arm 94% PCL-06% PGMA) | 0.5 | 3.5 | 315 | 35 | 1 |
| Example 3 (6 arm 96% PCL-04% PGMA) | 0.5 | 2.1 | 329 | 21 | 1 |

After dipentaerythritol (initiator, Sigma Aldrich) and hydroquinone (HQ, inhibitor, Sigma Aldrich) were placed in a three-necked flask and vacuum dried for 10 minutes, nitrogen purging was performed at a rate of 50 cc/min. Purified ε-caprolactone (ε -CL, monomer, AVENTION) was additionally added and stirred at 110°C for 10 minutes. Afterwards, glycidyl methacrylate (GMA, monomer, Sigma Aldrich) was added and stirred for 10 minutes, followed by injecting 1,5,7-triazabicyclo(4.4.0)dec-5-ene (TBD, catalyst, TCI) dissolved in acetonitrile (CAN, Sigma Aldrich) and and reacting at 110°C for 6 hours. After dissolving the final compound in chloroform (Daejung Chemicals & Metals CO., LTD.), it was precipitated in ethyl ether (Daejung Chemicals & Metals CO., LTD.) at 4°C, and vacuum dried after filtering.

The synthetic mechanism is as shown in FIG. 1, and when the monomers ε-caprolactone (ε -CL) and glycidyl methacrylate (GMA), the initiator dipentaerythritol and the inhibitor hydroquinone (HQ) are reacted at 110°C for 6 hours, the ring structure in the monomers is opened to synthesize polycaprolactone-polyglycidyl methacrylate (6-arm PCL-PGMA) having 6 arms.

### Comparative Example: Synthesis of 4-arm 94% PCL-06% PGMA

A 4-arm PCL-PGMA copolymer was synthesized as follows.

**[Table 2]**

| | **HD (mmol)** | **HQ (mmol)** | **ε-CL (mmol)** | **GMA (mmol)** | **TBD (mmol)** |
|---|---|---|---|---|---|
| Comparative Example (4 arm 94% PCL-06% PGMA) | 0.5 | 2.5 | 225 | 25 | 1 |

After pentaerythritol (initiator, Sigma Aldrich) and hydroquinone (HQ, inhibitor, Sigma Aldrich) were placed in a three-necked flask and vacuum dried for 10 minutes, nitrogen purging was performed at a rate of 50 cc/min. Purified ε-caprolactone (ε-CL, monomer, AVENTION) was additionally added and stirred at 110°C for 10 minutes. Afterwards, glycidyl methacrylate (GMA, monomer, Sigma Aldrich) was added and stirred for 10 minutes, followed by injecting 1,5,7-triazabicyclo(4.4.0)dec-5-ene (TBD, catalyst, TCI) dissolved in acetonitrile (CAN, Sigma Aldrich) and reacting at 110°C for 6 hours. The final compound was dissolved in chloroform (Daejung Chemicals & Metals CO., LTD.), precipitated in ethyl ether at 4°C (Daejung Chemicals & Metals CO., LTD.) and vacuum dried after filtering.

**<Experimental example>**

**Experimental Example 1: Cross-linking of copolymer compound**

100 w/v % of the synthesized copolymer compound and Irgacure2959 (photoinitiator, Sigma Aldrich) were added at a ratio of 1 w/v% and dissolved in *N*-methyl-2-pyrrolidone (NMP, Sigma Aldrich). After fabricating the dissolved solution in the form of a film, it was cross-linked using a UV lamp (365 nm/200 s).

**Experimental Example 2: Structural analysis**

Structural analysis was performed using $^1$H-NMR (AVANCE III HD 400, Bruker Biospin, USA). Samples were prepared using the copolymer compound synthesized in the above example and chloroform-D (Sigma Aldrich) at a concentration of 10 mg/mL.

The structural analysis results are shown in FIG. 2, and the PCL peaks were shown at $\delta$=4.10 [m, -$OCH_2$, (E)], 2.41 [m, -$CH_2$, (A)], 1.74 [m, -$CH_2$, (B and D)], 1.45 [m, -$CH_2$, (C)], and the PGMA peaks were shown at =6.13 [s, =$CH_2$, (G2)], 5.58 [s, =$CH_2$, (G1)], 1.97 [s, -$CH_3$, (F)], which confirmed that a compound having a 6-arm structure was synthesized. The mol% of PCL and PGMA was calculated by comparing the area of the PCL peak and the area of the PGMA peak.

**Experimental Example 3: Molecular weight analysis (GPC)**

The molecular weight of the compounds synthesized in the examples was analyzed using GPC (1260 Infinity II, Agilent). Samples were prepared with the synthesized compound and tetrahydrofuran (tetrahydrofuran, THF, J.T. Baker) at a concentration of 5 mg/mL and filtered with a 0.45 μm syringe filter. THF was used as the eluent, the flow rate was 1mL/min, and the column temperature was 40°C. The standard curve was prepared using PS (polystyrene, Agilent).

A GPC graph showing the molecular weight of the synthesized compound is shown in FIG. 3 and Table 3. The molecular weight of the 6-arm PCL-PGMA copolymer (Example) is shown to be higher than the molecular weight of the conventional 4-arm PCL-PGMA copolymer (Comparative Example), and it can be confirmed that in the case of the 6-arm PCL-MGA, as the PCL content increases, the molecular weight increases.

**[Table 3]**

| **Samples** | **Mn (*Da*)** | **Mw (*Da*)** | **PDI*** |
|---|---|---|---|
| Comparative Example (4 arm 94% PCL - 06% PGMA) | 6,918 | 9,864 | 1.43 |
| Example 1 (6 arm 92% PCL - 08% PGMA) | 6,938 | 9,167 | 1.32 |
| Example 2 (6 arm 94% PCL - 06% PGMA) | 7,253 | 10,403 | 1.43 |
| Example 3 (6 arm 96% PCL - 04% PGMA) | 12,940 | 21,930 | 1.69 |

**Experimental Example 4: Analysis of thermal properties (DSC)**

Thermal properties were analyzed using DSC (DSC214, NETZSCH). It was measured in the temperature range of -80°C to 150°C in the heating-cooling-heating mode, and the heating and cooling rate was carried out at 10°C/min.

The results of measuring DSC before cross-linking are shown in FIG. 4 and Table 4. When comparing 94% PCL-06% PGMA of the conventional 4-arm structure as a comparative example and 6-arm 94% PCL-06% PGMA of Example 2, the melting temperature (Tm), melting enthalpy ($\Delta_m$) and crystallization enthalpy ($\Delta_c$) of the example were shown to be low compared to the comparative example. This means that in the case of 6-arm PCL-PGMA, which is the example, the proportion of PGMA increased because the number of terminal groups increased by increasing the number of arms compared to the comparative example. Due to the increased proportion of PGMA, the crystal formation of PCL was more hindered, and as a result, the examples showed different thermal properties from the comparative examples.

In the case of Examples 1, 2 and 3, it can be confirmed that the melting temperature, melting enthalpy and crystallization enthalpy increased as the proportion of PCL, which is a crystalline polymer, increased, and this is also because changes in the proportion of PGMA affected the crystal formation of PCL.

[Table 4]

| Samples | $T_{m,1}$ (°C) | $T_{m,2}$ (°C) | $\Delta H_m$ (J/g) | $T_c$ (°C) | $\Delta H_c$ (J/g) |
|---|---|---|---|---|---|
| Comparative Example (4 arm 94% PCL - 06% PGMA) | 44.16 | 49.73 | 65.70 | 19.22 | 65.37 |
| Example 1 (6 arm 92% PCL - 08% PGMA) | 35.20 | 44.20 | 51.37 | 4.7 | 53.64 |
| Example 2 (6 arm 94% PCL - 06% PGMA) | 42.70 | 49.20 | 62.41 | 14.50 | 63.71 |
| Example 3 (6 arm 96% PCL - 04% PGMA) | 45.80 | 51.70 | 71.39 | 16.80 | 71.27 |

The results of measuring DSC after cross-linking are shown in FIG. 5 and Table 5. By cross-linking the synthesized copolymer compound, polymer chains are connected, and through this, shape-memory performance appears, which affects the melting temperature (shape restoration temperature) after cross-linking. In addition, crystallinity is reduced by cross-linking the synthesized compound having a 6-arm type structure, which results in a melting temperature lower than that before cross-linking (comparison of FIGS. 4 and 5).

In the case of Example 1, since the content of PGMA participating in the cross-linking reaction was high, the melting temperature and crystallization temperature did not appear because it became amorphous while cross-linking. In the case of Examples 2 and 3, the degree of crystallinity could be controlled by adjusting the ratio of PCL and PGMA, and the change in crystallinity affected the melting temperature. The shape restoration temperature can be controlled by adjusting the ratio of PCL and PGMA in the 6-arm structured PCL-PGMA copolymer which is synthesized using these properties.

[Table 5]

| Samples | $T_{m,}$ (°C) | $\Delta H_m$ (J/g) | $T_c$ (°C) | $\Delta H_c$ (J/g) |
|---|---|---|---|---|
| Comparative Example (4 arm 94% PCL - 06% PGMA) | 37.60 | 38.73 | -2.80 | 35.80 |
| Example 1 (6 arm 92% PCL - 08% PGMA) | - | - | - | - |
| Example 2 (6 arm 94% PCL - 06% PGMA) | 40.30 | 47.85 | 7.40 | 47.61 |
| Example 3 (6 arm 96% PCL - 04% PGMA) | 46.30 | 63.15 | 22.70 | 63.61 |

**Experimental Example 5: Analysis of mechanical properties (UTM)**

The mechanical properties of the shape-memory polymer of the present invention were analyzed using UTM (34SC-1, INSTRON). The samples were UV cross-linked to fabricate in the form of a film having a size of W 5 mm × L 5 mm × T 0.4 mm, and it was measured under the conditions of the initial length of 10 mm and the tensile speed of 10 mm/min at 37°C.

The mechanical properties through UTM measurement are shown in FIG. 6 and Table 6. Young's modulus (E), stress at max ($\sigma$) and strain ($\varepsilon$) values were increased in the examples compared to the comparative examples. Particularly, in the case of Example 2, Young's modulus (E) increased by 812%, stress at max ($\sigma$) increased by 383%, and strain ($\varepsilon$) increased by 189% compared to the comparative example. These results were obtained because chain entanglement increased as the number of arms increased in the copolymer structure, resulting in increased mechanical properties and increased PCL content, thereby increasing crystallinity.

[Table 6]

| Samples | *E* (MPa) | $\sigma$ (MPa) | $\varepsilon$ (%) |
|---|---|---|---|
| Comparative Example (4 arm 94% PCL - 06% PGMA) | 10.14 ± 0.90 | 3.31 ± 0.43 | 556.57 ± 0.90 |
| Example 2 (6 arm 94% PCL - 06% PGMA) | 29.48 ± 2.35 | 6.54 ± 1.30 | 602.04 ± 18.76 |
| Example 3 (6 arm 96% PCL - 04% PGMA) | 82.40 ± 13.47 | 12.69 ± 0.72 | 1054.44 ± 35.10 |

Experimental Example 6: Analysis of shape-memory properties (DMA)

Shape-memory properties were analyzed using DMA (Discovery DMA850, TA instrument). The sample was

UV cross-linked to produce a film having a size of W 5 mm × L5 mm × T 0.4 mm.

After maintaining at 55°C for 10 minutes (original permanent shape, $\varepsilon_p(0)$), strain was applied to deform the sample by stretching to 78 kPa at a rate of 4 kPa/min. Afterwards, it was cooled at a rate of 2°C/min to 0°C, maintained at 0°C for 10 minutes (maximum strain, $\varepsilon_1(N)$), and then released at a rate of 4 kPa/min to 0Pa (temporary shape, $\varepsilon_u(N)$), and then, the temperature was raised to 55°C at a rate of 2°C/min (permanent shape, $\varepsilon_p(N)$). This process was repeated a total of 4 times as one cycle.

The shape recovery rate ($R_r$) and the shape fixation degree ($R_f$) were calculated according to Formulas (1) and (2) below.

$$R_r(N) = \frac{\varepsilon_1(N)-\varepsilon_p(N)}{\varepsilon_1(N)-\varepsilon_p(N-1)} \times 100\% \quad (1)$$

$$R_f(N) = \frac{\varepsilon_u(N)}{\varepsilon_1(N)} \times 100\% \quad (2)$$

The shape-memory properties of the copolymer-based shape-memory polymer of Example 2 through DMA measurement are shown in FIG. 7 and Table 7. When the 6-arm PCL-PGMA copolymer was synthesized and then cross-linked, shape-memory properties were exhibited, and specifically, in order to create a deformed shape of the 6-arm PCL-PGMA-based shape-memory polymer, after the shape-memory polymer was deformed at a temperature higher than the shape restoration temperature, the temperature was lowered to 0°C or lower and fixed, and when the temperature was raised again, it was confirmed that the original shape was restored. Even when the cycle was tested by repeating 4 times using this method, all showed shape-memory properties. As a result of measurements, the average shape fixation ability was 105.01%, and it was confirmed that the average shape restoration ability was 91.46%. Meanwhile, the results of photographing the shape-memory properties are shown in FIG. 8, and it was confirmed that 100% shape restoration was achieved in all example-based shape-memory polymers.

**[Table 7]**

| | **Number of cycles** | | | |
|---|---|---|---|---|
| | 1 | 2 | 3 | 4 |
| **$R_r$ (%)** | 64.06 | 104.60 | 98.36 | 98.85 |
| **$R_f$ (%)** | 103.71 | 103.99 | 106.12 | 106.24 |

$R_r$: shape recovery; $R_f$: shape fixity; %: strain relaxation ratio compared to original length.

**Experimental Example 7: Design and fabrication of vascular outer wall support**

According to the physical properties of the compound according to the present invention and the shape-memory properties by cross-linking, the shape-memory polymer according to the present invention is a biocompatible material which is capable of restoring the shape by temperature control, and it can be developed as a medical material such as a vascular outer wall support.

Accordingly, a vascular outer wall support prepared by the shape-memory polymer according to the present invention was designed. In an aspect, the vascular outer wall support may be provided to surround the implanted autologous veins and arteries as a side-to-end model during arteriovenous fistula formation, and in this case, the vascular outer wall support can help the maturation of transplanted autologous veins, minimize the occurrence of abnormal blood flow, and facilitate the flow of blood.

Therefore, the vascular outer wall support according to the present invention is designed to include an arterial support part and a transplanted vein support part, and in order to minimize the occurrence of abnormal blood flow of

blood vessels flowing from arterial blood to venous blood when the vascular outer wall support anastomoses blood vessels, an anastomosis device was designed in a U shape, and the curvature was presented and compared according to the angle of the vein junction.

The areas where vascular stenosis is likely to occur during arteriovenous fistula formation are known as the toe, heel and floor areas based on the anastomosis, and the analysis was conducted based on these areas. However, in the case of the floor area, based on the tangent line where the vein contacts the artery, the proximal area mainly plays an important role in arteriovenous fistula blood flow, and thus, the analysis was conducted with the proximal area of the tangent line as the floor area.

As a result of analyzing a change in blood flow according to the angle of the vein anastomosis, it was possible to maintain blood flow in a U shape, which induces blood flow that is hemodynamically closer to laminar flow than connecting blood vessels with conventional surgical methods, and it was expected to prevent neointimal formation and be able to prevent vascular stenosis. As the curvature was lower, the results were obtained in which the blood flow, became stable, but realistically, when the anatomical structures were reflected, it was determined that it would be particularly desirable to form a radius of curvature of 10 to 20 mm (about 30 to 40°) (FIGS. 9 and 10).

Based on the above results, a vascular blood vessel support including a transplanted vein support part and an artery support part was designed and fabricated (FIGS. 11 and 12).

The vascular outer wall support fabricated as described above was stretched in the circumferential direction using a UTM (34SC-1, INSTRON) to measure the circumferential tensile strength. At 37°C, the initial length was 3 mm, and the tensile rate was measured at a rate of 10 mm/min.

As a result, compared to the vascular outer wall support fabricated by the shape-memory polymer based on Comparative Example (4 arm 94%PCL-06%PGMA), it was confirmed that the vascular outer wall support fabricated by the shape-memory polymer based on Example 2 (6 arm 94%PCL-06%PGMA) had a certain tendency and the circumferential tensile strength increased by about 7% on average (FIG. 13).

Meanwhile, the pressurized bursting strength of the fabricated vascular outer wall support was measured using the pressurized bursting strength measuring device of FIG. 14a. The pressurized bursting strength measuring device consists of a pressure gauge, a syringe pump and a sample holder, and it is designed to measure the pressure that can withstand when a balloon is inserted into the sample and then inflated by injecting water into the balloon at a constant rate. The vein part of the vascular outer wall support was mounted on the pressurized bursting strength measuring device, and the bursting pressure when the inner tube was expanded was determined. The injection rate of distilled water at 37°C was measured at a rate of 10 cc/min.

As a result, compared to the vascular outer wall support fabricated by the shape-memory polymer based on the comparative example (4arm 94%PCL-06%PGMA), it was confirmed that the vascular outer wall support fabricated by the shape-memory polymer based on Example 2 (6arm 94%PCL-06%PGMA) had a certain tendency and the pressurized bursting strength increased by about 39% on average (FIG. 14).

### Experimental Example 8. Structural change of the venous vessel walls according to the application of the vascular outer wall support through computer modeling

In order to determine the effect of the vascular outer wall support according to the present invention, structural changes were determined through computer modeling, when veins were wrapped with the vascular outer wall support.

Vein is a biological tissue with hyperelastic properties, and since it does not follow the linear and nonlinear properties of other polymer materials, it recovers elastically and exhibits incompressible behavior even when considerable plastic deformation occurs.

In the present invention, parameters were calculated to simulate a homogeneous, isotropic and incompressible hyperelastic model for blood vessels.

The strain energy function (W) is expressed as a function of the elongation invariant and a function of the principal elongation, and is defined as Formula (3).

$$W = W(I1,\ I2,\ I3) \tag{3}$$

Since most elastic bodies are incompressible, I3 = 1, and Formula (3) can be derived as Formula (4).

$$W = W(I1, I2) \quad (4)$$

I1 and I2 are defined as invariants of principal elongation (λ), and the 2-curve fitting analysis was performed for the rabbit inferior vena cava using the 2-parameter Mooney Rivlin model which is applied to the deformation analysis of incompressible elastic bodies.

The Mooney-Rivlin 2 parameter model is defined as Formula (5).

$$G_{Mooney} = 2(C_{10} + C_{01}) \quad (5)$$

In this case, the values of C10 and C01 are constant terms and are derived during curve fitting, and the values of C10 = 0.01 MPa and C01 = 0.0095 MPa were obtained using ANSYS Software. When the vascular outer wall support was applied to the artery-vein-artery straight model by applying the calculated physical properties, it was determined by modeling what changes occurred in the vascular outer wall support and the vessel wall.

After connecting the vein and artery, arterial pressure was applied to the vein, and the vein was deformed by the pressure. When the arterial pressure was 120 mmHg, which was the maximum systolic pressure, changes in the anatomical structure of the inner diameter of the vessel occurred. However, compared to a case where the blood vessel diameter increased nearly three times when only veins were present, it was confirmed that the vascular outer wall support prevents the rapid expansion of blood vessels, and the bridge structure can further alleviate the changes.

**Experimental Example 9: Validation using animal model**

Animal experiments were conducted in compliance with the guidelines of IACUC (KBIO-IACUC-2021-174) of the Osong Advanced Medical Industry Promotion Foundation (KBIO Health). Zolazepam (5 mg/kg, Zoletil™, Virbac Korea) and xylazine (2 mg/kg, Rompun® Bayer Korea) were injected intramuscularly to 7 to 8 kg female beagles (Orient Bio) to anesthetize, and the anesthetized state was maintained during the surgical time by respiratory anesthesia (isoflurane, <2.5 %, Piramal).

An arteriovenous fistula (AVF) animal model using a dog was prepared as follows (FIG. 16).

A midline incision (about 5 cm) of the inner thigh of the right hindlimb of beagles (n = 6/group) was made to locate the femoral artery and vein under the connective tissue. After tying the distal part of the femoral vein with 4-0 silk and cutting the blood vessel, blood flow was temporarily blocked using a vascular tournique in the proximal and distal parts of the femoral artery. A longitudinal incision of about 5 mm was made on the side surface of the femoral artery, and an end-to-side anastomosis was performed with the previously incised vein using 7-0 prolene. In the experimental group (w/vascular outer wall support) with the vessel outer wall support (fabricated by the shape-memory polymer crosslinked in Example 2) inserted, the anastomosis was performed after the vascular outer wall support was placed in the venous area before the vein and artery anastomosis, and the artery part was treated with saline solution in the body temperature range, and the support was fixed by surrounding the artery. Afterwards, the subcutaneous layer and skin were closed to complete model production. In the control group where no vascular outer wall support was added (w/o vascular outer wall support), all steps except the step of adding the vascular outer wall support were performed as is.

After 3 months and 6 months of arteriovenous fistula modeling, the blood flow pattern of the arteriovenous anastomosis site was determined using ultrasonography (EKO7, Samsung Medison). As a result, in the case of the anastomosis vessels of the control group without the vascular outer wall support, vortex formation intensified over time, whereas the vortex formation of the experimental group with the vascular outer wall support inserted was suppressed, and the flow of arterial blood (red arrow) appeared smoothly (FIG. 17).

After 6 months of arteriovenous fistula modeling, the condition and patency of the arteriovenous anastomosis were determined by angiography. A catheter (6Fr) was placed in the iliac artery, and iohexol was injected to perform angiography using a C-arm. As a result, as shown in FIG. 18, it was confirmed that the anastomosis state was well maintained in a U shape and had good patency (FIG. 18).

Finally, after sacrificing the subject by the intravenous injection of potassium chloride under general anesthesia, the arteriovenous anastomosis site was collected to perform histopathology. The collected blood vessels were fixed by treatment with 4% paraformaldehyde for 24 hours, and then embedded in paraffin wax, and the arteriovenous anastomosis site was cross-sectioned to a thickness of 5 μm. As a result of pentachrome staining (Pentachrome staining, StatLab,

item #: KTRMP) of the cut cross-section, it was confirmed that compared to the control group, the effect of inhibiting neointima formation was excellent in the experimental group where the vascular outer wall support according to the present invention was inserted (FIG. 19).

Having described specific parts of the content of the present invention in detail above, it will be clear to those skilled in the art that these specific descriptions are only preferred exemplary embodiments, and the scope of the present invention is not limited thereto. Accordingly, the substantial scope of the present invention will be defined by the appended claims and their equivalents.

## Claims

1. A compound represented by Chemical Formula (1) below:

Chemical Formula (1)

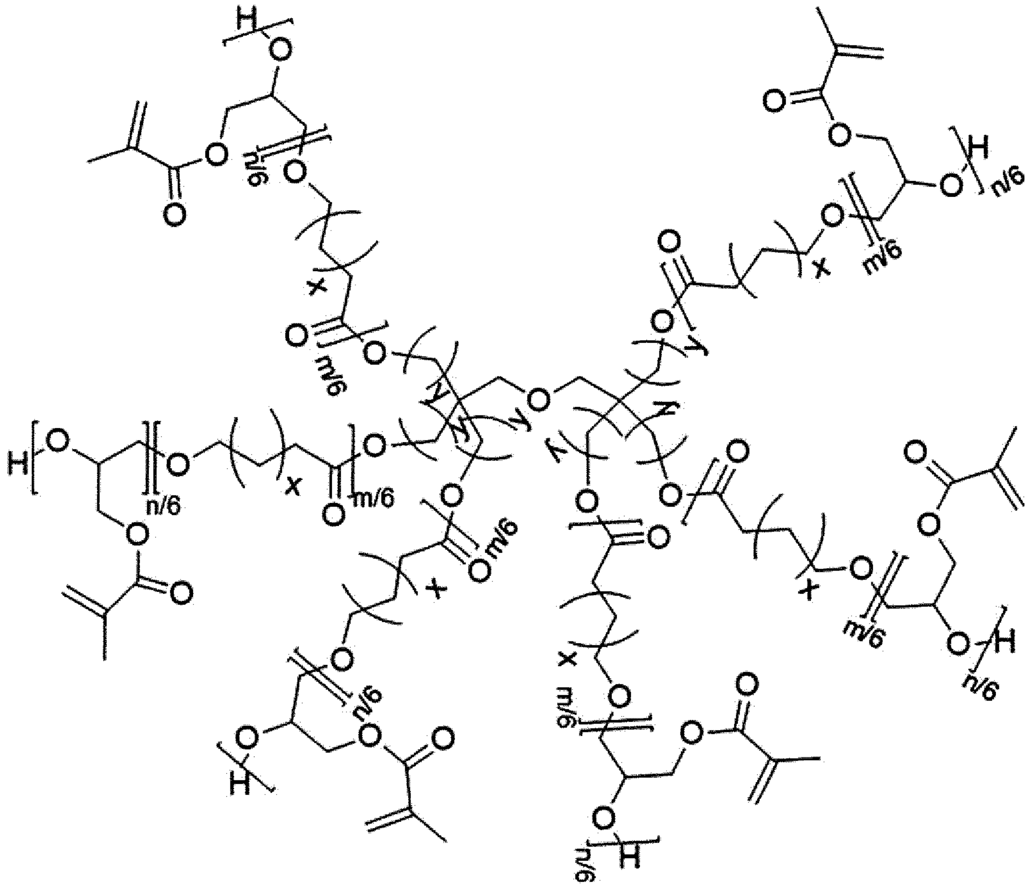

wherein in Chemical Formula (1) above, x and y are each independently integers from 1 to 20,
wherein m and n represent mol% of repeat units, and
wherein m + n is 100, and m is 70 to 99.

2. The compound of claim 1, wherein the compound has shape-memory properties by cross-linking.

3. The compound of claim 1, wherein the compound has an average shape restoration ability of 90% or more at a temperature of 35 to 58°C after cross-linking.

4. A method for preparing the compound of claim 1, comprising the step of:
reacting dipentaerythritol, caprolactone and glycidyl methacrylate.

5. The method of claim 4, wherein the reaction is a ring-opening polymerization reaction.

6. The method of claim 4, wherein the reaction is reacted under the presence of a catalyst selected from the group consisting of 1,5,7-triazabicyclo(4.4.0)dec-5-ene, tin(II) (2-ethylhexanoate), trimethylopropane tris(3-mercaptopropionate) and zinc succinate.

7. The method of claim 4, wherein the reaction is performed at 80 to 140°C.

8. A shape-memory polymer in which the compound of claim 1 is cross-linked.

9. A method for preparing a shape-memory polymer, further comprising the step of:
inducing a cross-linking reaction in the compound of claim 1 prepared by the method according to any one of claims 4 to 7.

10. The method of claim 9, wherein the cross-linking reaction is a photo-cross-linking or thermal cross-linking reaction.

11. A medical material, comprising the compound according to any one of claims 1 to 3.

12. The medical material of claim 11, wherein the medical material is a support for vascular transplantation or a stent for vascular transplantation.

13. The medical material of claim 12, wherein the support for vascular transplantation is a vascular outer wall support which is provided to surround transplanted autologous veins and arteries as a side-to-end model during arteriovenous fistula formation.

14. The medical material of claim 13, wherein the vascular outer wall support is a U-shaped anastomosis device.

15. The medical material of claim 13, wherein the vascular outer wall support comprises an artery support and a graft vein support, and the angle of a tangent at the anastomosis site between an artery and a vein is 30° to 90°.

【FIG. 1】

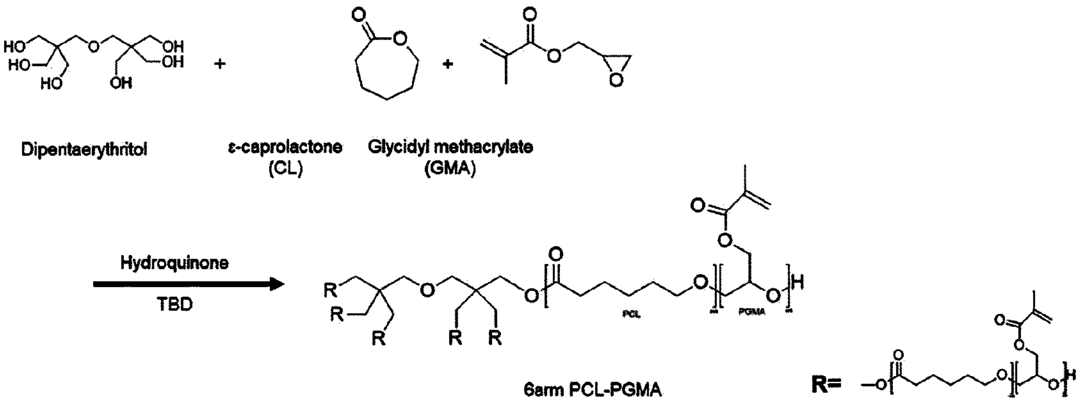
Dipentaerythritol
ε-caprolactone
(CL)
Glycidyl methacrylate
(GMA)
Hydroquinone
TBD
6arm PCL-PGMA

【FIG. 2】

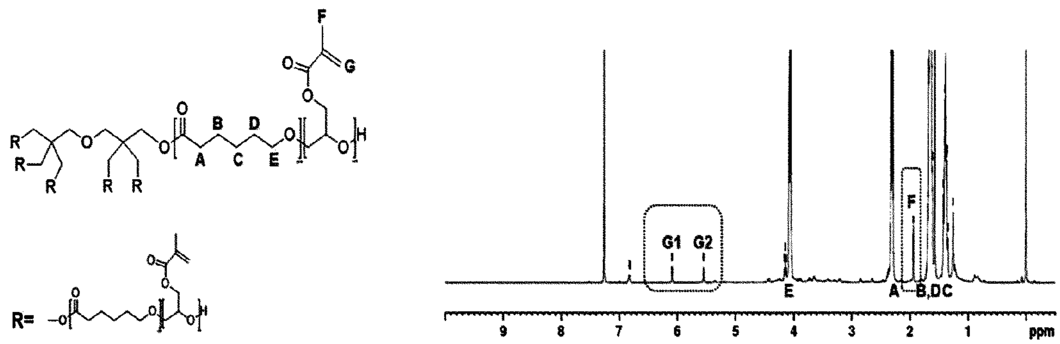
G1
G2
F
E
A
B,DC
ppm
9
8
7
6
5
4
3
2
1

【FIG. 3】

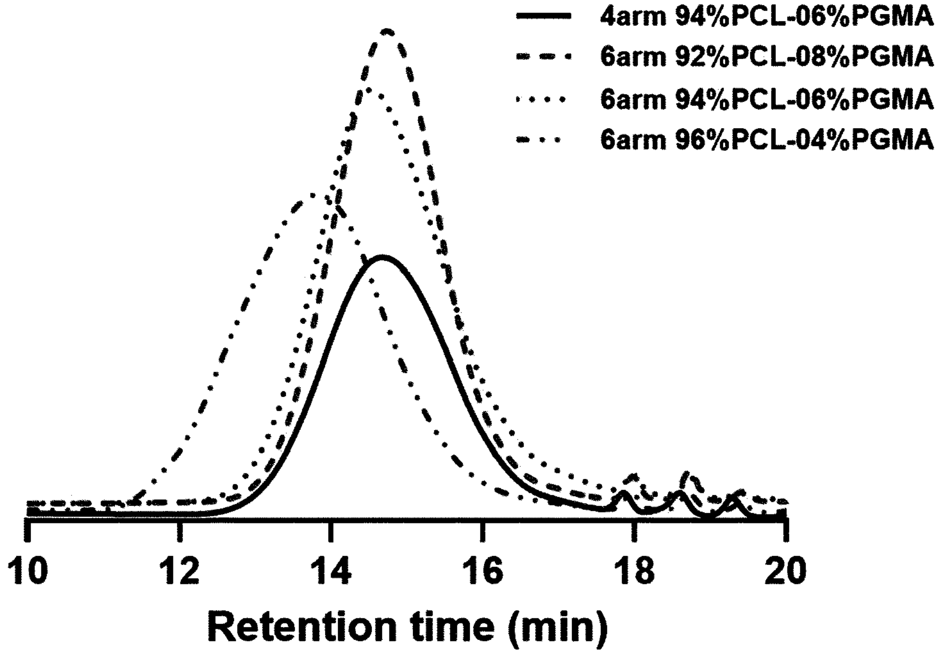
4arm 94%PCL-06%PGMA
6arm 92%PCL-08%PGMA
6arm 94%PCL-06%PGMA
6arm 96%PCL-04%PGMA
10
12
14
16
18
20
Retention time (min)

【FIG. 4a】

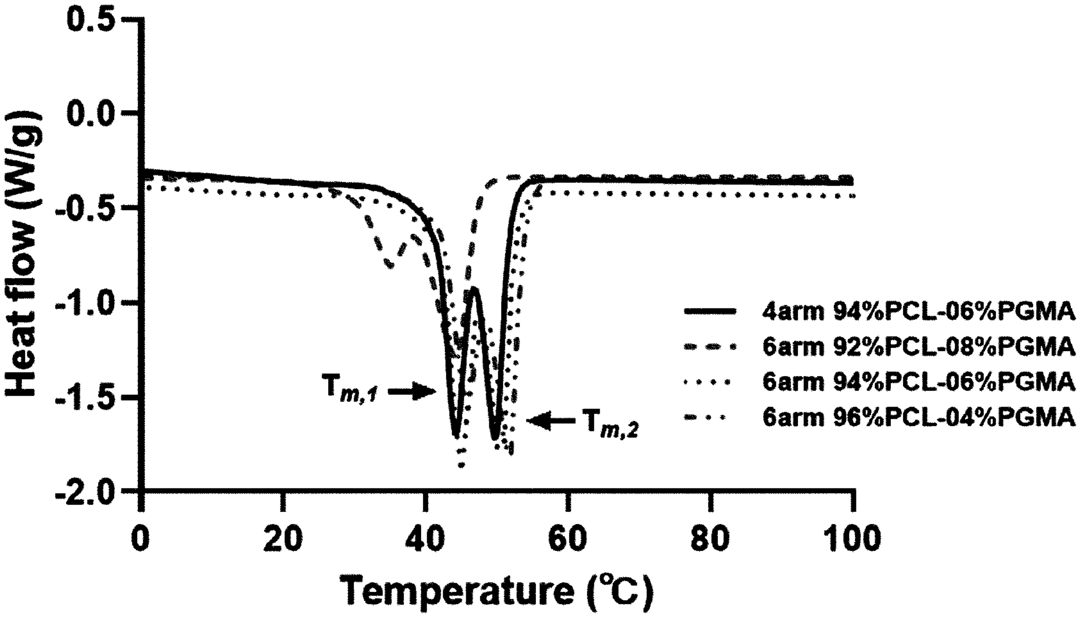
Heat flow (W/g)
Temperature (℃)
Tm,1
Tm,2
4arm 94%PCL-06%PGMA
6arm 92%PCL-08%PGMA
6arm 94%PCL-06%PGMA
6arm 96%PCL-04%PGMA

【FIG. 4b】

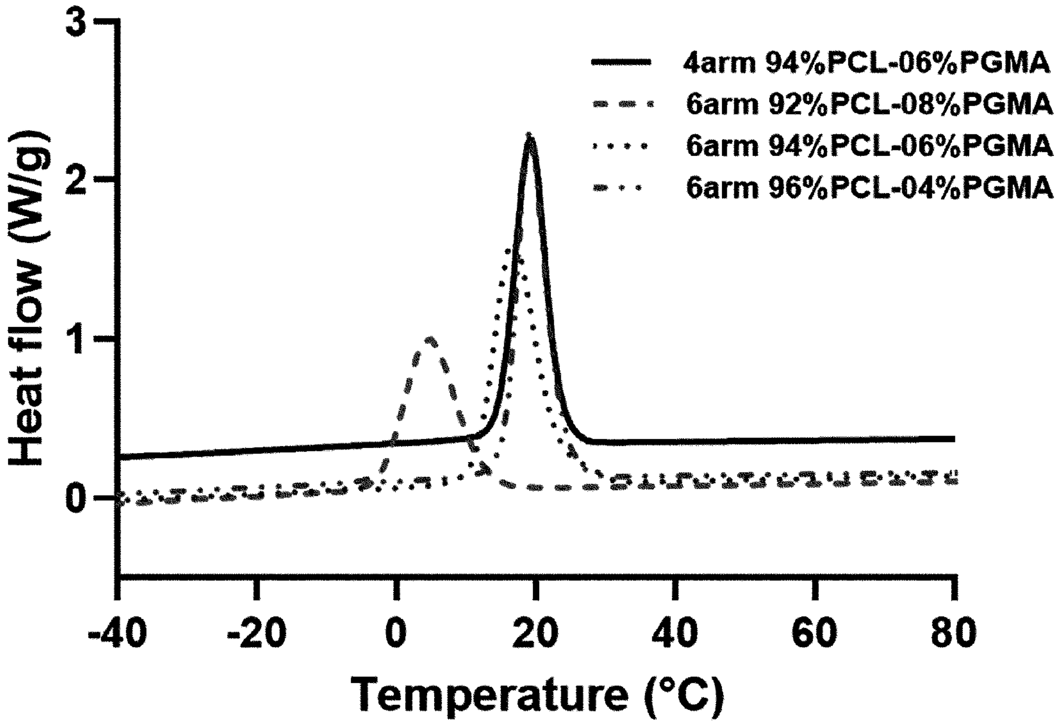
Heat flow (W/g)
Temperature (°C)
4arm 94%PCL-06%PGMA
6arm 92%PCL-08%PGMA
6arm 94%PCL-06%PGMA
6arm 96%PCL-04%PGMA

【FIG. 5a】

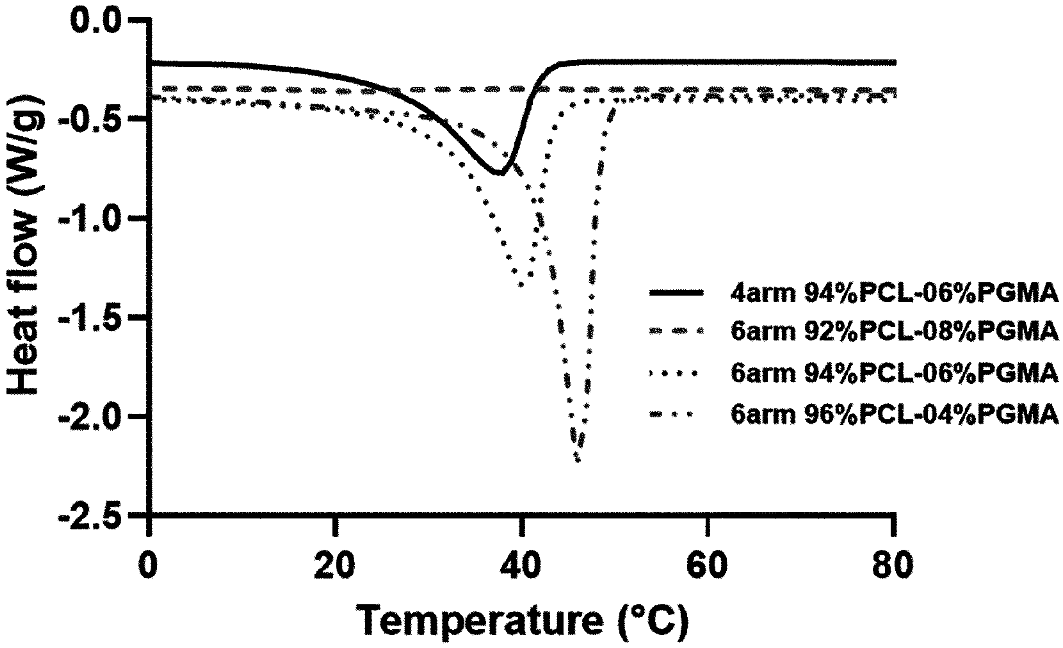
0.0
-0.5
-1.0
-1.5
-2.0
-2.5
Heat flow (W/g)
0
20
40
60
80
Temperature (°C)
4arm 94%PCL-06%PGMA
6arm 92%PCL-08%PGMA
6arm 94%PCL-06%PGMA
6arm 96%PCL-04%PGMA

【FIG. 5b】

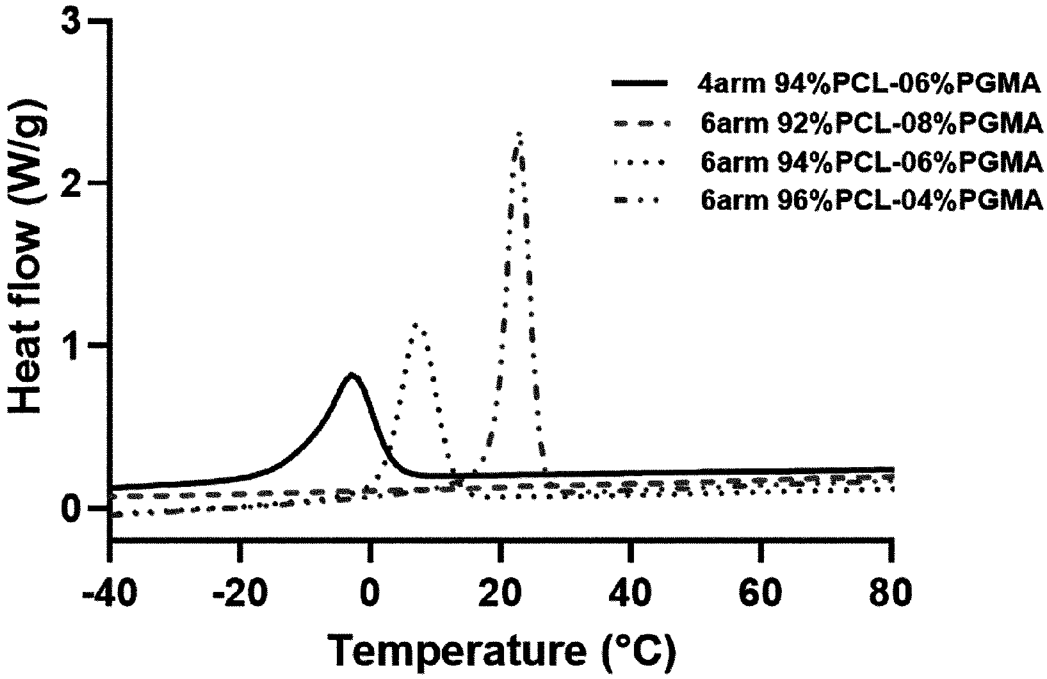
3
2
1
0
Heat flow (W/g)
-40
-20
0
20
40
60
80
Temperature (°C)
4arm 94%PCL-06%PGMA
6arm 92%PCL-08%PGMA
6arm 94%PCL-06%PGMA
6arm 96%PCL-04%PGMA

【FIG. 6】

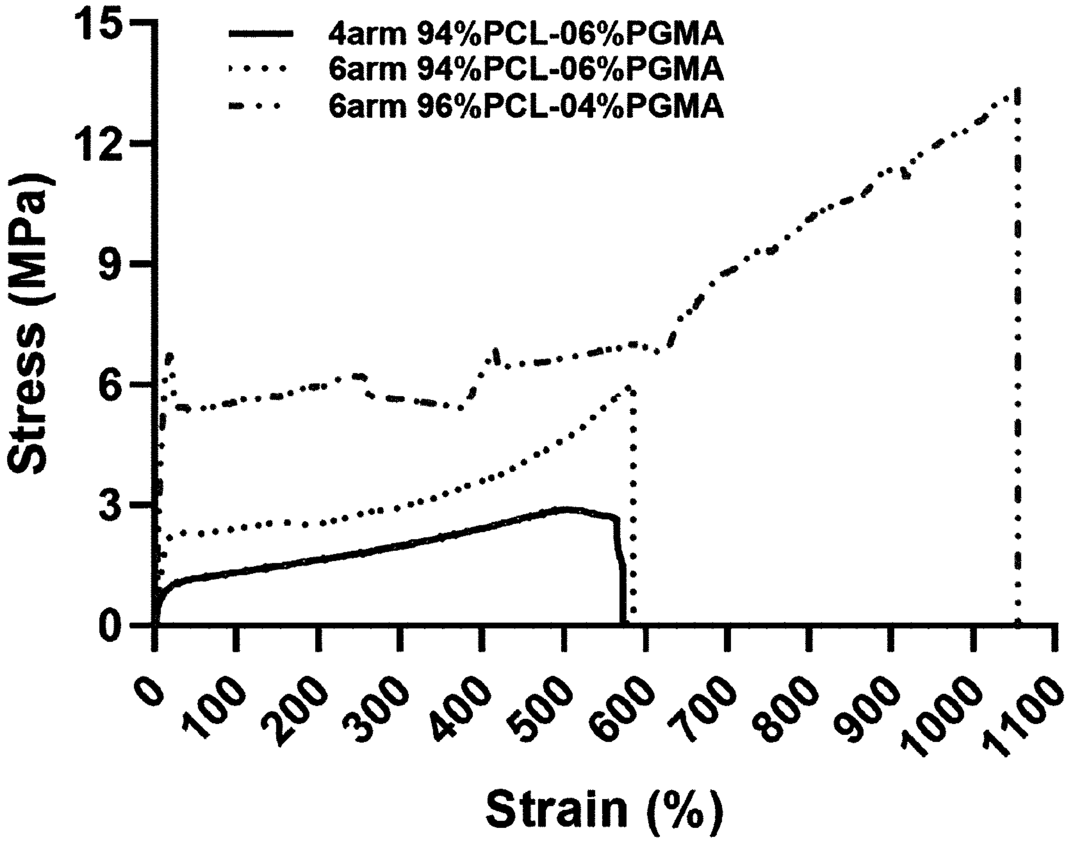
15
12
9
6
3
0
Stress (MPa)
4arm 94%PCL-06%PGMA
6arm 94%PCL-06%PGMA
6arm 96%PCL-04%PGMA
0
100
200
300
400
500
600
700
800
900
1000
1100
Strain (%)

【FIG. 7】

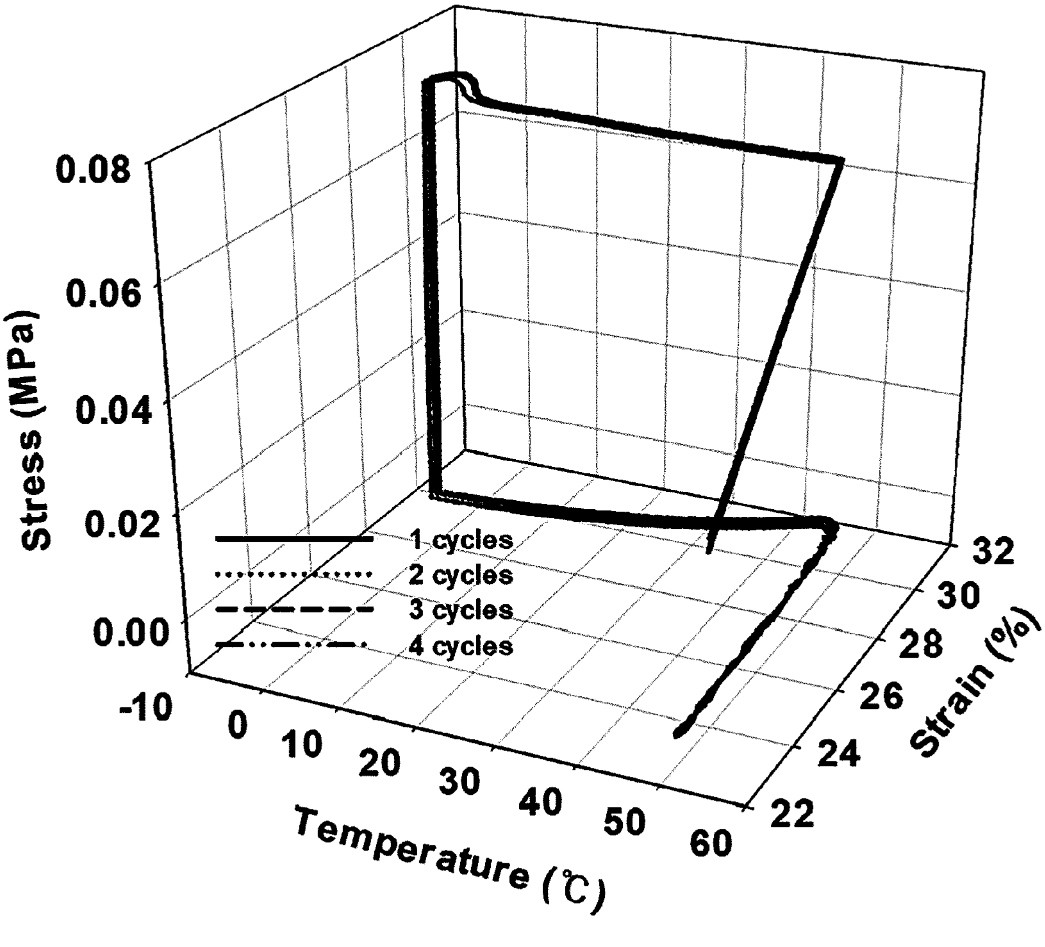
0.08
0.06
0.04
0.02
0.00
Stress (MPa)
1 cycles
2 cycles
3 cycles
4 cycles
-10
0
10
20
30
40
50
60
Temperature (℃)
22
24
26
28
30
32
Strain (%)

【FIG. 8】

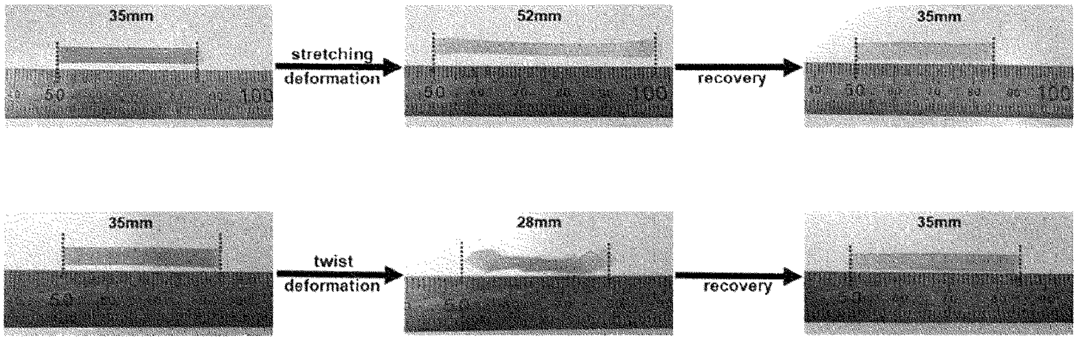
35mm
stretching deformation
52mm
recovery
35mm
35mm
twist deformation
28mm
recovery
35mm

【FIG. 9】

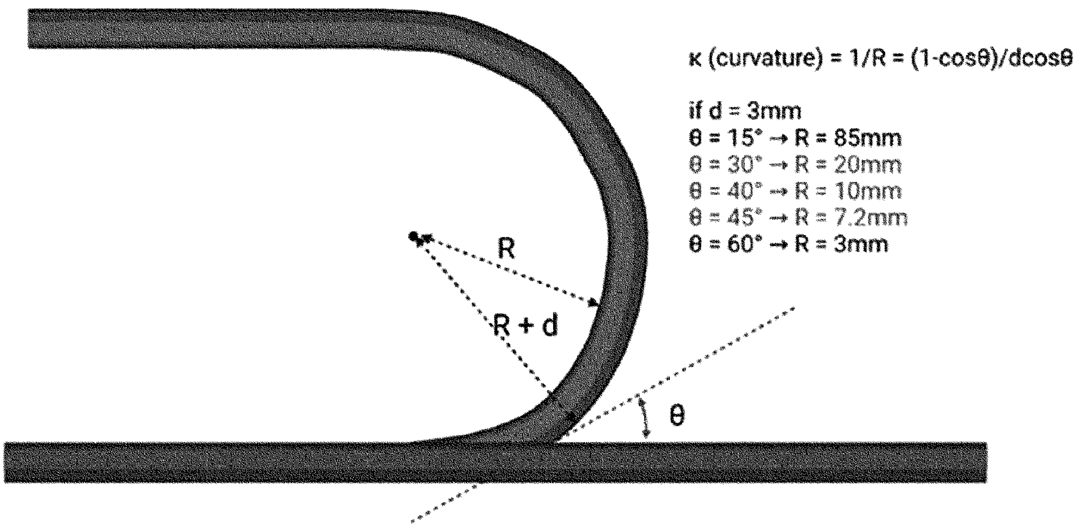
κ (curvature) = 1/R = (1-cosθ)/dcosθ
if d = 3mm
θ = 15° → R = 85mm
θ = 30° → R = 20mm
θ = 40° → R = 10mm
θ = 45° → R = 7.2mm
θ = 60° → R = 3mm
R
R + d
θ

【FIG. 10a】

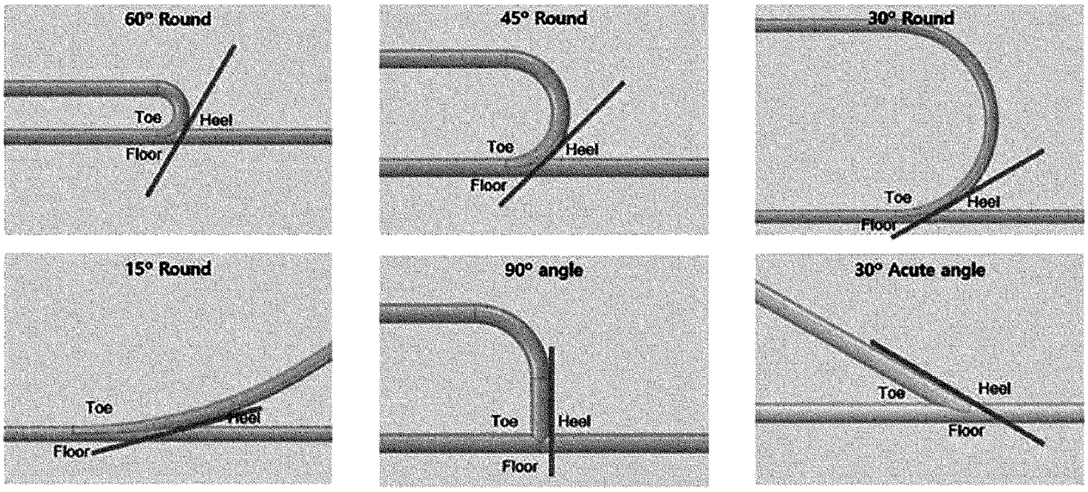


【FIG. 10b】

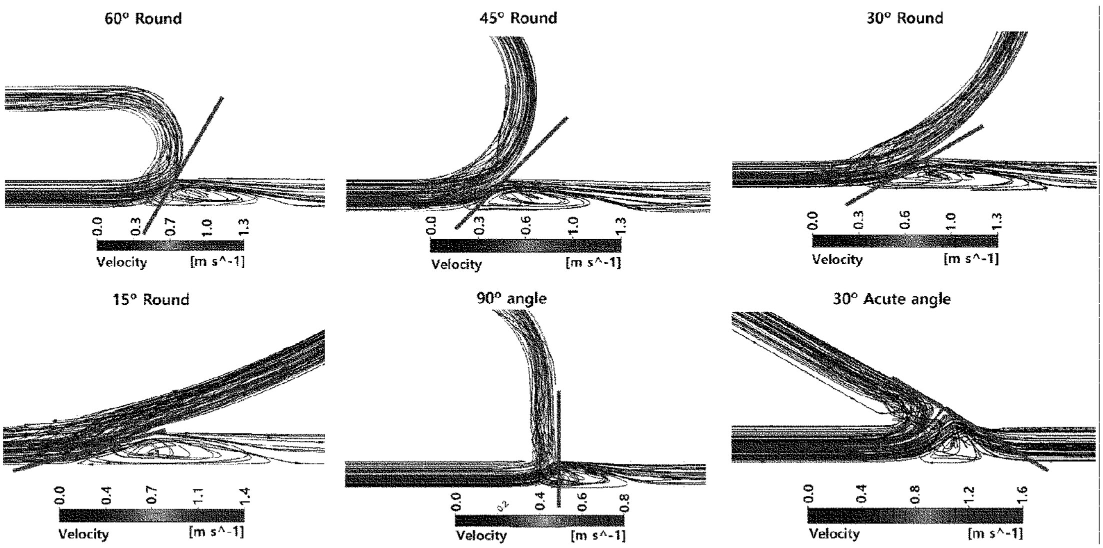

【FIG. 11a】

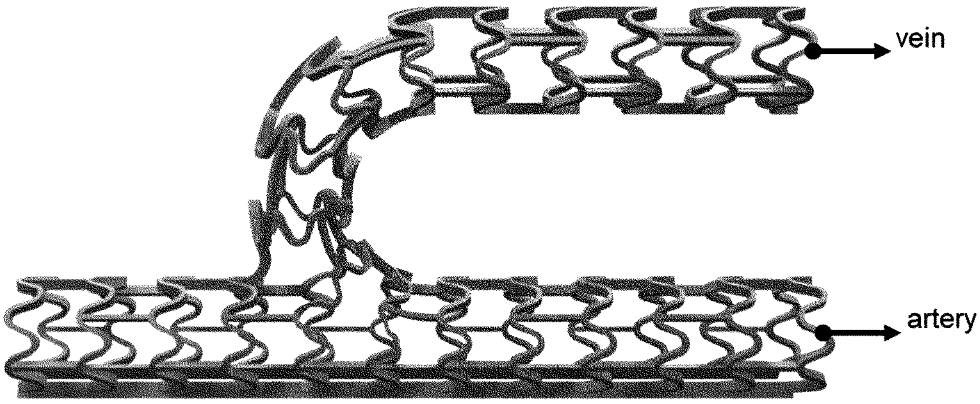


【FIG. 11b】

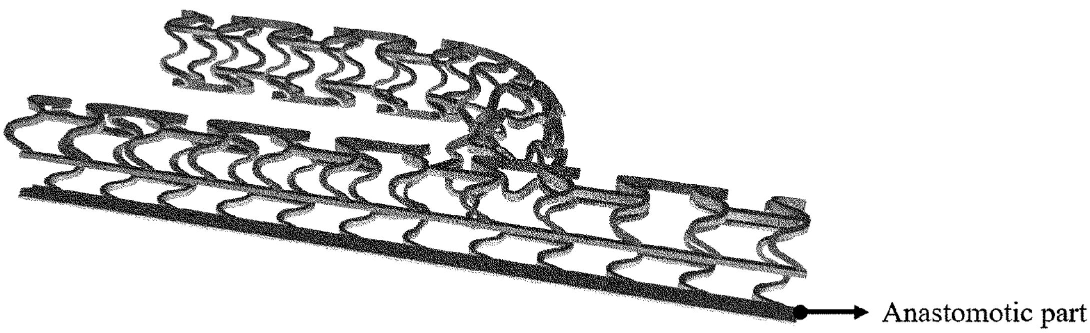

【FIG. 12】

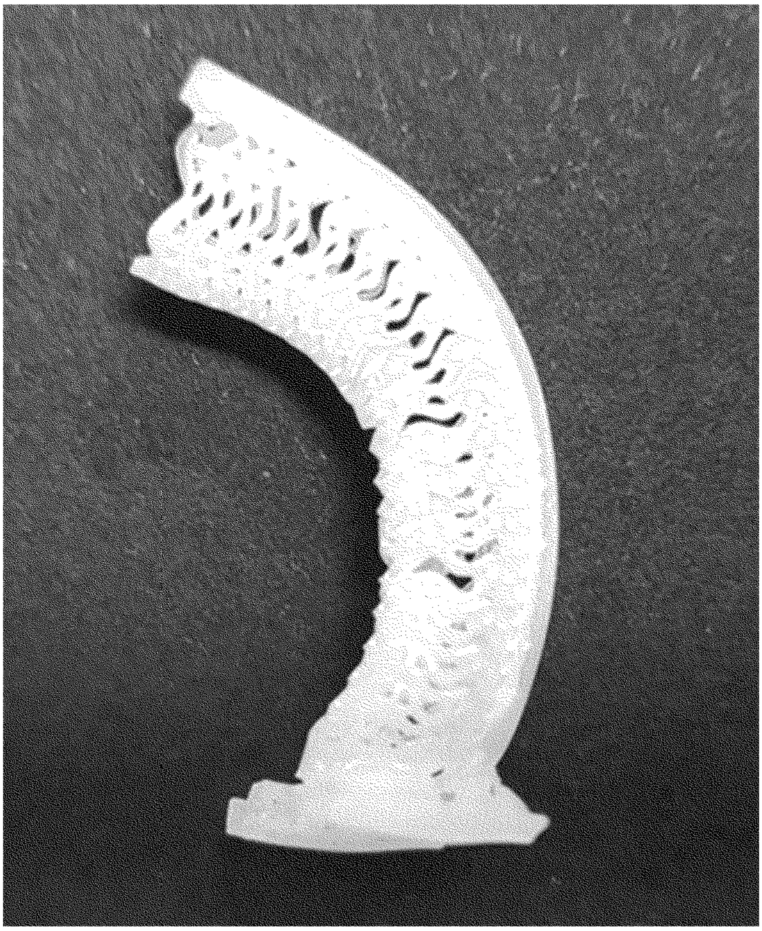

【FIG. 13】

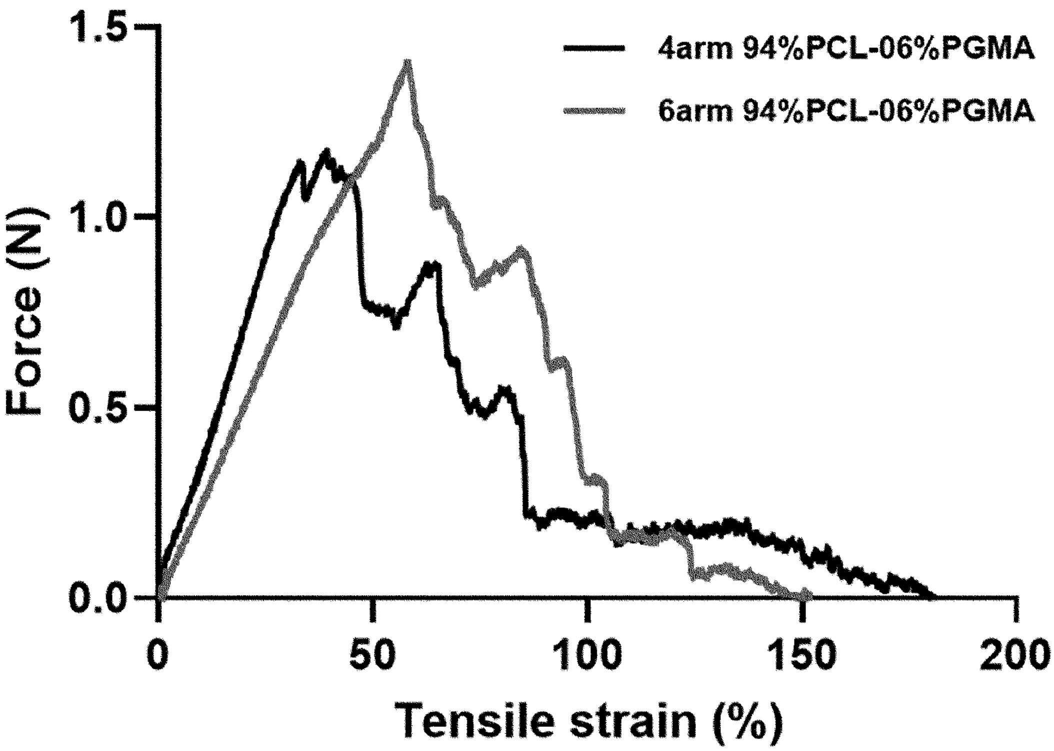
4arm 94%PCL-06%PGMA
6arm 94%PCL-06%PGMA
Force (N)
Tensile strain (%)
1.5
1.0
0.5
0.0
0
50
100
150
200

【FIG. 14a】

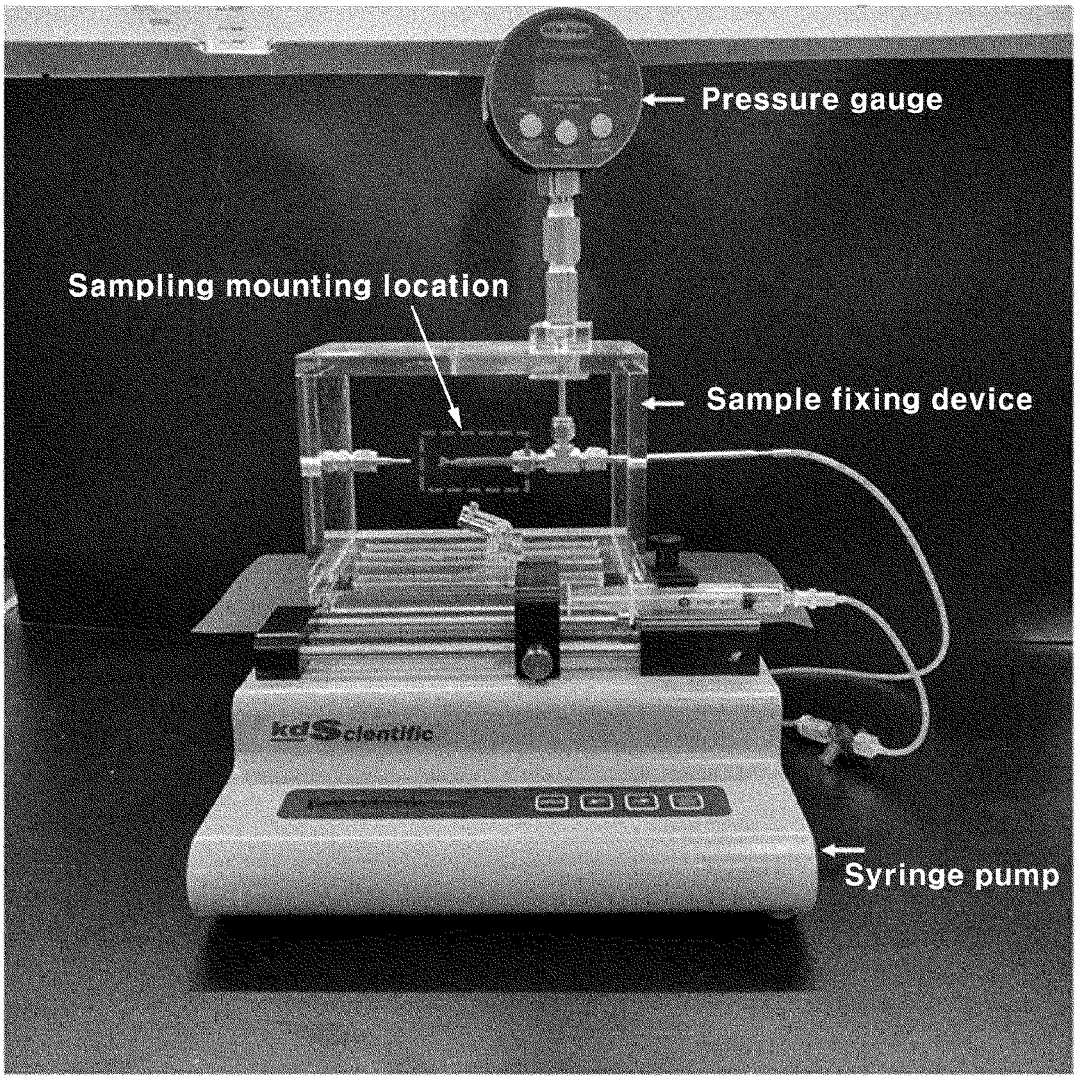
Pressure gauge
Sampling mounting location
Sample fixing device
Syringe pump

【FIG. 14b】

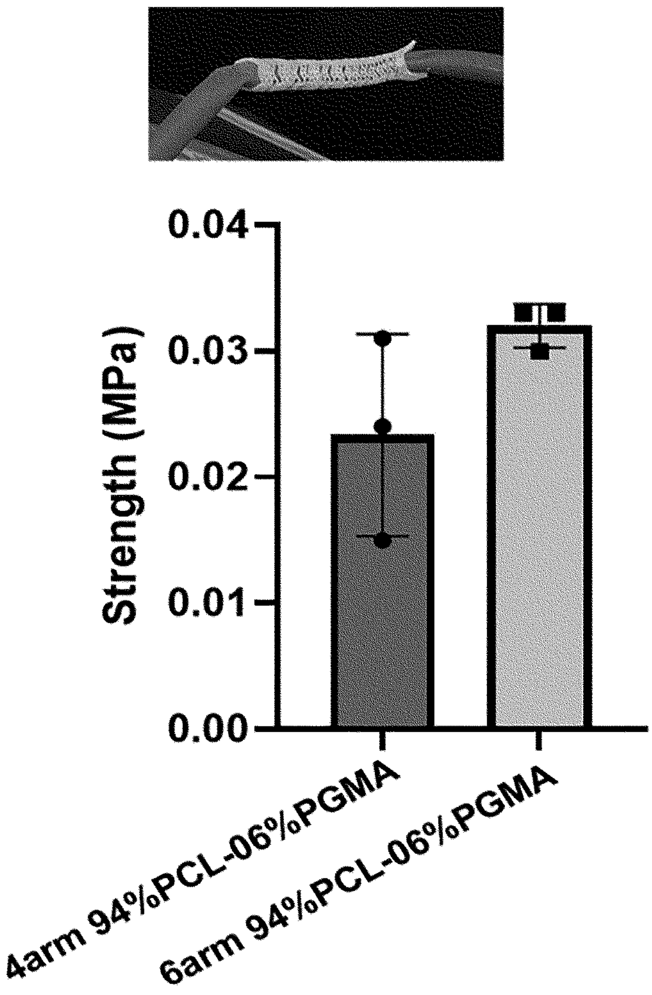
0.04
0.03
0.02
0.01
0.00
Strength (MPa)
4arm 94%PCL-06%PGMA
6arm 94%PCL-06%PGMA

【FIG. 15】

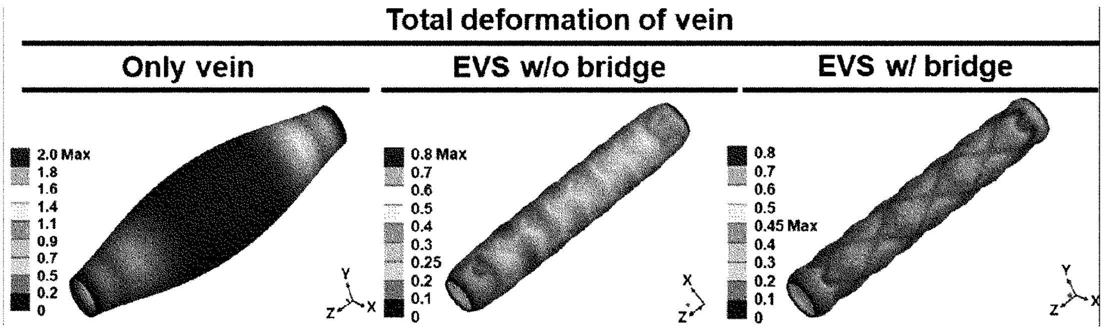
Total deformation of vein
Only vein
EVS w/o bridge
EVS w/ bridge
2.0 Max
1.8
1.6
1.4
1.1
0.9
0.7
0.5
0.2
0
0.8 Max
0.7
0.6
0.5
0.4
0.3
0.25
0.2
0.1
0
0.8
0.7
0.6
0.5
0.45 Max
0.4
0.3
0.2
0.1
0

【FIG. 16】

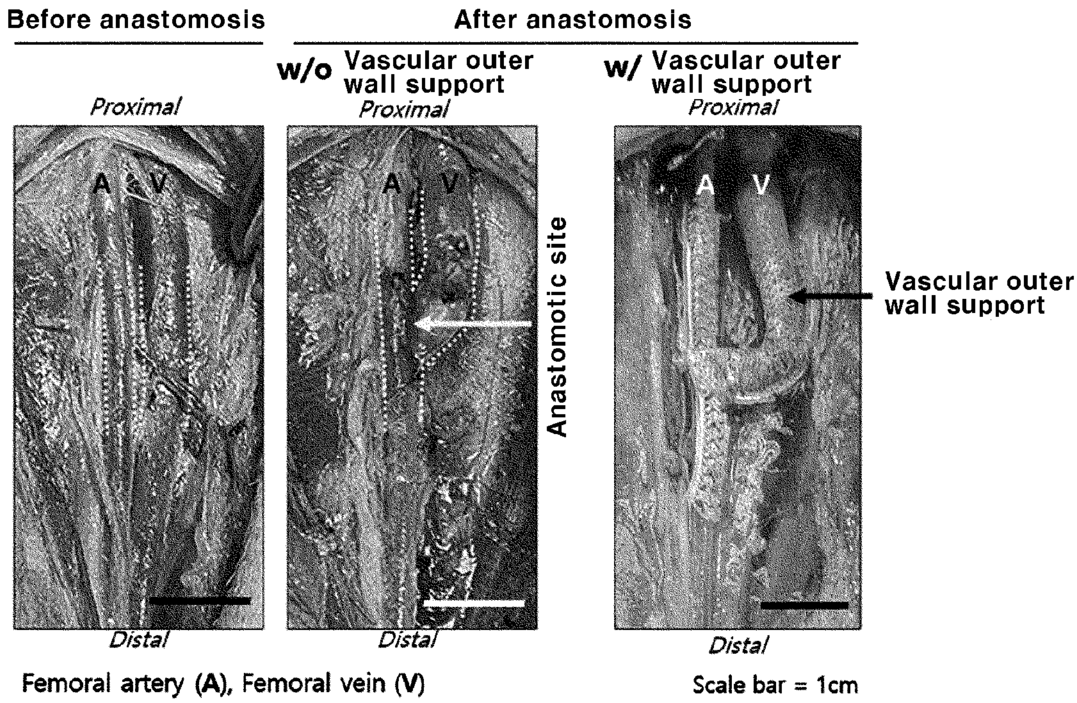
Before anastomosis
After anastomosis
w/o Vascular outer wall support
w/ Vascular outer wall support
Proximal
Proximal
Proximal
A
V
A
V
A
V
Anastomotic site
Vascular outer wall support
Distal
Distal
Distal
Femoral artery (A), Femoral vein (V)
Scale bar = 1cm

【FIG. 17】

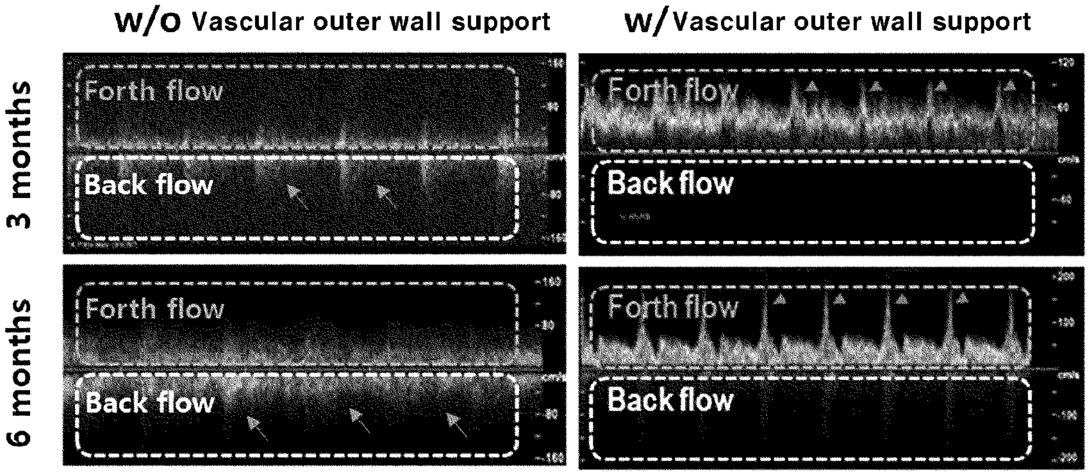


【FIG. 18】

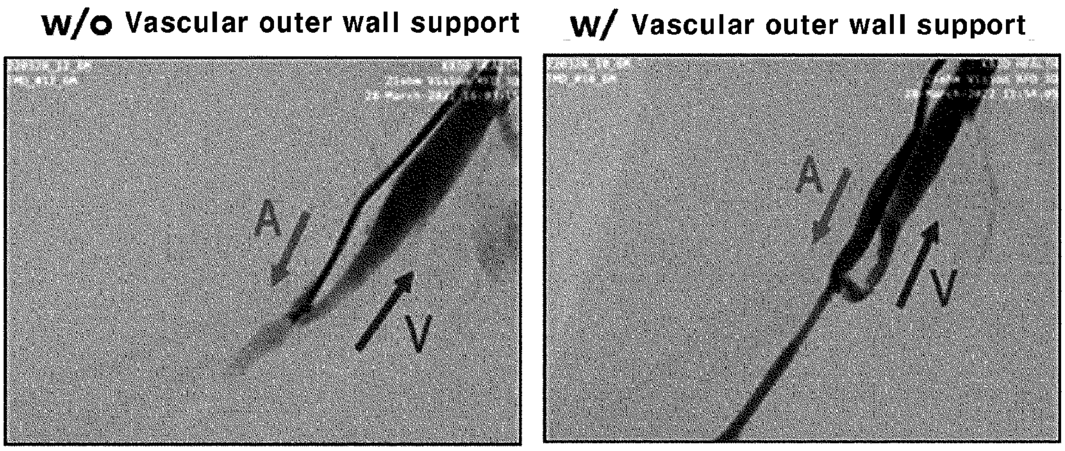


Femoral artery (**A**), Femoral vein (**V**)

【FIG. 19】

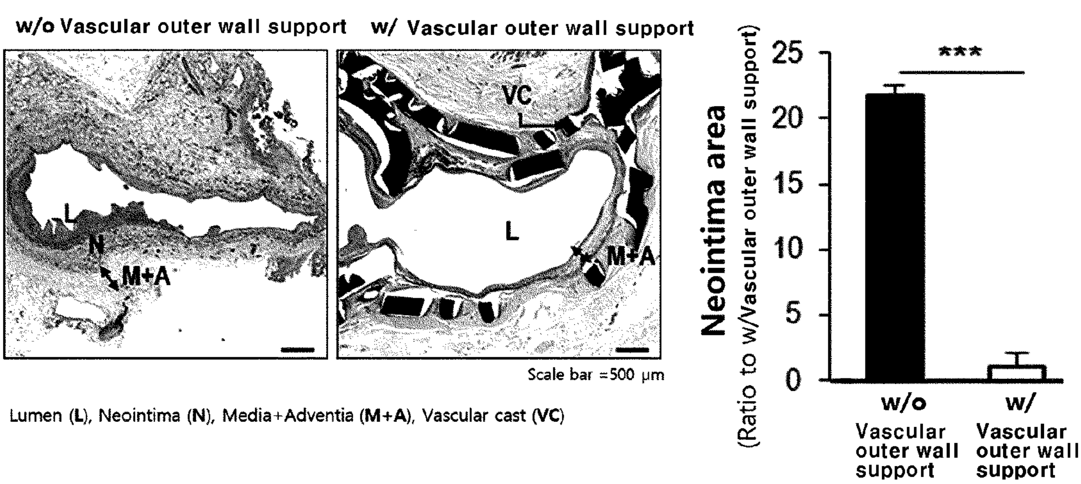
w/o Vascular outer wall support
w/ Vascular outer wall support
L
N
M+A
VC
L
L
M+A
Scale bar =500 μm
Lumen (L), Neointima (N), Media+Adventia (M+A), Vascular cast (VC)
Neointima area
(Ratio to w/Vascular outer wall support)
25
20
15
10
5
0
***
w/o
Vascular
outer wall
support
w/
Vascular
outer wall
support

## INTERNATIONAL SEARCH REPORT

International application No.
**PCT/KR2022/018588**

**A. CLASSIFICATION OF SUBJECT MATTER**

**C08G 83/00**(2006.01)i; **C08F 220/32**(2006.01)i; **A61L 27/14**(2006.01)i

According to International Patent Classification (IPC) or to both national classification and IPC

**B. FIELDS SEARCHED**

Minimum documentation searched (classification system followed by classification symbols)

C08G 83/00(2006.01); A61B 17/00(2006.01); C08F 220/18(2006.01); C08F 299/04(2006.01); C08G 18/42(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 형상기억(shape-memory), 디펜타에리쓰리톨(dipentaerythritol), 글리시딜메틸아크릴산(glycidyl methacrylate, GMA), ε-카프로락톤(ε-caprolactone)

**C. DOCUMENTS CONSIDERED TO BE RELEVANT**

| Category* | Citation of document, with indication, where appropriate, of the relevant passages | Relevant to claim No. |
|---|---|---|
| DA | KR 10-1906472 B1 (INDUSTRY-ACADEMIC COOPERATION FOUNDATION, YONSEI UNIVERSITY) 10 October 2018 (2018-10-10)<br>See claims 1-13; and paragraphs [0097], [0138], [0140], [0141], [0143]-[0149], [0151], [0164]-[0167], [0169] and [0171]-[0173]. | 1-15 |
| A | YANG, X. et al. Triple shape memory effect of star-shaped polyurethane. ACS Applied materials & interfaces. 2014, vol. 6, pp. 6545-6554.<br>See abstract; and formula 1. | 1-15 |
| A | DEFIZE, T. et al. Reversible TAD chemistry as a convenient tool for the design of (re)processable PCL-based shape-memory materials. Macromolecular rapid communications. 2017, vol. 38, thesis no.: 1600517, inner pp. 1-7.<br>See inner pages 1 and 2. | 1-15 |
| A | KR 10-2020-0038198 A (INDUSTRY-ACADEMIC COOPERATION FOUNDATION, YONSEI UNIVERSITY) 10 April 2020 (2020-04-10)<br>See claims 1-17. | 1-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 March 2023** | **08 March 2023** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**

International application No.
**PCT/KR2022/018588**

**C. DOCUMENTS CONSIDERED TO BE RELEVANT**

| Category* | Citation of document, with indication, where appropriate, of the relevant passages | Relevant to claim No. |
|---|---|---|
| A | CN 112778481 A (CHINA PETROLEUM & CHEMICAL CORP. et al.) 11 May 2021 (2021-05-11) See entire document. | 1-15 |

Form PCT/ISA/210 (second sheet) (July 2022)

**INTERNATIONAL SEARCH REPORT**
**Information on patent family members**

International application No.
**PCT/KR2022/018588**

| Patent document cited in search report | Publication date (day/month/year) | Patent family member(s) | Publication date (day/month/year) |
|---|---|---|---|
| KR 10-1906472 B1 | 10 October 2018 | EP 3608346 A1 | 12 February 2020 |
| | | EP 3608346 B1 | 09 March 2022 |
| | | ES 2916412 T3 | 30 June 2022 |
| | | US 2021-0102023 A1 | 08 April 2021 |
| | | WO 2018-186575 A1 | 11 October 2018 |
| KR 10-2020-0038198 A | 10 April 2020 | JP 2022-508632 A | 19 January 2022 |
| | | KR 10-2355542 B1 | 26 January 2022 |
| | | US 2022-0000481 A1 | 06 January 2022 |
| | | WO 2020-071806 A1 | 09 April 2020 |
| CN 112778481 A | 11 May 2021 | CN 112778481 B | 12 July 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 101906472
- KR 102208921